(19)

**Europäisches Patentamt**
**European Patent Office**
**Office européen des brevets**

(11) **EP 2 933 744 B1**

(12) **EUROPEAN PATENT SPECIFICATION**

(45) Date of publication and mention
of the grant of the patent:
**12.10.2022 Bulletin 2022/41**

(51) International Patent Classification (IPC):
***G16C 20/30*** *(2019.01)*

(21) Application number: **15163451.6**

(52) Cooperative Patent Classification (CPC):
**G16C 20/30**

(22) Date of filing: **14.04.2015**

(54) **SIMULATION METHOD FOR HIGH-POLYMER MATERIAL**

SIMULATIONSVERFAHREN FÜR HOCHPOLYMERMATERIAL

PROCÉDÉ DE SIMULATION POUR MATÉRIAU DE HAUT POLYMÈRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.04.2014 JP 2014086665
14.11.2014 JP 2014231208**

(43) Date of publication of application:
**21.10.2015 Bulletin 2015/43**

(73) Proprietor: **Sumitomo Rubber Industries, Ltd.
Kobe-shi, Hyogo 651-0072 (JP)**

(72) Inventor: **Ueno, Shinichi
Kobe-shi, Hyogo 651-0072 (JP)**

(74) Representative: **Manitz Finsterwald
Patent- und Rechtsanwaltspartnerschaft mbB
Martin-Greif-Strasse 1
80336 München (DE)**

(56) References cited:
**EP-A1- 2 672 405**

• **Starr Francis W. ET AL: "Molecular Dynamics
Simulation of a Polymer Melt with a Nanoscopic
Particle", Macromolecules, vol. 35, no. 11, 21 May
2002 (2002-05-21), pages 4481-4492,
XP055800535, Washington DC United States
ISSN: 0024-9297, DOI: 10.1021/ma010626p
Retrieved from the Internet:
URL:https://pubs.acs.org/doi/pdf/10.1021/m
a010626p>**

**Description**

Technical **Field**

The present invention relates to a simulation method for a high-polymer material which is useful for estimating and improving the dispersion of filler.

Background Art

In a high-polymer material (e.g. rubber) containing filler (e.g. carbon black, silica and the like), the strength is largely affected by the dispersion of the filler.

In recent years, in order to improve and develop compositions of rubber compounds, there have been proposed various computer-implemented numerical simulation methods for estimating the dispersion of filler contained in a high-polymer material .

In this kind of numerical simulation method, a numerical model of the filler is defined by a plurality of particles (hereinafter "F-particle"), and a numerical model of the high-polymer material is defined by a plurality of particles (hereinafter "P-particle"). Then, the P-particles are bound with the F-particles, and a molecular dynamics calculation is performed by a computer.

EP 2 672 405 A1 discloses a method for simulating the dispersion of fillers comprising a filler model defining step, a polymer model defining step and executing a molecular dynamics calculation by using the filler models and the polymer models.

Summary of invention

Technical Problem

In the above-described simulation method, P-particles to be bound with each F-particle are randomly selected by a computer. Consequently, the number of the P-particles bound with one F-particle varies largely. If the number becomes large, as the total force of the P-particles exerted on the F-particle becomes large, the molecular dynamics calculation has a high probability of causing a calculation failure.

It is therefore, a primary object of the present invention to provide a simulation method for a high-polymer material in which, a calculation failure during a molecular dynamics calculation is efficiency prevented by limiting the number of P-particles to be bound with each F-particle not to exceed a predetermined upper limit value.

Solution to Problem

According to one aspect of the present invention, a simulation method for a high-polymer material is a computer-implemented method for estimating the dispersion of filler contained in the high-polymer material, and comprises:

a process for defining filler models of the filler, wherein each filler model is a numerical model made up of a plurality of F-particles,
a process for defining polymer models of the high-polymer material, wherein each polymer models is a numerical model made up of a plurality of P-particles, and
a simulation process for executing a molecular dynamics calculation by the use of the filler models and the polymer models disposed in a predetermined virtual space,
the simulation process including a bonding process for binding bonding-target P-particles selected from the P-particles with bonding-target F-particles selected from the F-particles, wherein the number of the bonding-target P-particle(s) to be bound with each bonding-target F-particle satisfies a predetermined upper limit value $Su$, wherein the upper limit value $Su$, the total number $Na$ of the bonding-target P-particles existing in the virtual space, and the total number $Nb$ of the bonding-target F-particles existing in the virtual space satisfy the following expression:

$$Na/Nb-1 =< Su =< Na/Nb+1.$$

Here, the term "F-particle" means a particle of a filler model. The term "P-particle" means a particle of a polymer model.

The upper limit value $Su$ satisfies the following expression:

$$Na/Nb-1 =< Su =< Na/Nb+1$$

wherein

Na is a total number of the bonding-target P-particles existing in the virtual space, and
Nb is a total number of the bonding-target F-particles existing in the virtual space.

The bonding process may include:

a process for defining the upper limit value Su,
a selecting process for selecting the bonding-target P-particle(s) to be bound with each bonding-target F-particle, and
a process for binding the selected bonding-target P-particle(s) with the concerned bonding-target F-particle.

The selecting process may include:

a 1st process for associating each bonding-target P-particle with a bonding-target F-particle to which interparticle distance is smallest,
a 2nd process for selecting the bonding-target P-particle(s) to be bound with each bonding-target F-particle from a group of the bonding-target P-particle(s) associated with the concerned bonding-target F-particle in the ascending order of the interparticle distance to satisfy the upper limit value Su,
a third process for associating each bonding-target P-particle not selected in the 2nd process with a bonding-target F-particle to which interparticle distance is second smallest next to said bonding-target F-particle to which interparticle distance is smallest, and
a fourth process for selecting the bonding-target P-particle(s) to be bound with each bonding-target F-particle from a group of the bonding-target P-particle(s) associated in the third process, in the ascending order of the interparticle distance to satisfy the upper limit value Su, and
the third process and the fourth process are repeated until all of the bonding-target P-particles are selected for the bonding-target F-particles.

Preferably, the F-particles of each filler model include those disposed at apexes of a polyhedron, and the bonding-target F-particles are the F-particles disposed at the apexes.

According to another aspect of the present invention, a simulation method for a high-polymer material is a computer-implemented method for estimating the dispersion of filler contained in the high-polymer material together with a coupling agent for coupling the filler with polymer by the use of a computer, comprises:

a process for defining filler models of the filler, wherein each filler model is a numerical model made up of a plurality of F-particles,
a process for defining polymer models of the high-polymer material, wherein each polymer models is a numerical model made up of a plurality of P-particles, and
a process for defining coupling agent models of the coupling agent, wherein each coupling agent model is a numerical model made up of at least one C-particle,
a simulation process for executing a molecular dynamics calculation by the use of the filler models, the coupling agent models and the polymer models disposed in a predetermined virtual space,
the simulation process including a bonding process in which bonding-target P-particles selected from the P-particles and bonding-target C-particles selected from the C-particles are bound with bonding-target F-particles selected from the F-particles, wherein
the number of the bonding-target P-particle(s) and/or the bonding-target C-particle(s) to be bound with each bonding-target F-particle satisfies a predetermined upper limit value Ns
wherein the upper limit value Ns , the total number Na of the bonding-target P-particles existing in the virtual space, the total number Nb of the bonding-target F-particles existing in the virtual space, and the total number Nc of the bonding-target C-particles existing in the virtual space satisfy the following expression:

$$Ns = [(Na+Nc)/Nb+1].$$

Here, The term "F-particle" means a particle of a filler model. The term "P-particle" means a particle of a polymer model. The term "C-particle" means a particle of a coupling agent model.

The upper limit value Ns satisfies the following expression:

$$Ns = [(Na+Nc)/Nb+1]$$

wherein

  Na is a total number of the bonding-target P-particles existing in the virtual space,
  Nb is a total number of the bonding-target F-particles existing in the virtual space, and
  Nc is a total number of the bonding-target C-particles existing in the virtual space.

The bonding process may include:

  a process for defining the upper limit value Ns,
  a selecting process for selecting the bonding-target P-particle(s) and/or the bonding-target C-particle(s) to be bound with each bonding-target F-particle, and
  a process for binding the selected bonding-target P-particle(s) and/or bonding-target C-particle(s) with the concerned bonding-target F-particle.

The selecting process may include:

  a 1st process for associating each bonding-target P-particle with a bonding-target F-particle to which interparticle distance is smallest, and associating each bonding-target C-particle with a bonding-target F-particle to which interparticle distance is smallest,
  a 2nd process for selecting the bonding-target P-particle(s) and/or the bonding-target C-particle(s) to be bound with each bonding-target F-particle from the associated bonding-target P-particle(s) and/or bonding-target C-particle(s) in the ascending order of the interparticle distance to satisfy the upper limit value Ns,
  a third process for associating each bonding-target P-particle not selected in the 2nd process with a bonding-target F-particle to which interparticle distance is second smallest next to said bonding-target F-particle to which interparticle distance is smallest, and
  associating each bonding-target C-particle not selected in the 2nd process with a bonding-target F-particle to which interparticle distance is second smallest next to said bonding-target F-particle to which interparticle distance is smallest, and
  a fourth process for selecting the bonding-target P-particle(s) and/or the bonding-target C-particle(s) to be bound with each bonding-target F-particle from the bonding-target P-particle(s) and/or the bonding-target C-particle(s) associated in the third process in the ascending order of the interparticle distance to satisfy the upper limit value Ns, and
  the third process and the fourth process are repeated until all of the bonding-target P-particles and the bonding-target C-particles are selected for the bonding-target F-particles.

Preferably, the F-particles of each filler model include those disposed at apexes of a polyhedron, and the bonding-target F-particles are the F-particles disposed at the apexes.

According to the present invention, the number of bonding-target P-particle(s) and/or C-particle(s) to be bound with each bonding-target F-particle does not exceed the predetermined upper limit value to prevent an excessive increase in the summation of forces of the bonding-target P-particle(s) and/or C-particle(s) exerted on each bonding-target F-particle, therefore, it is possible to perform the molecular dynamics calculation stably without causing calculation failure.

Brief Description of Drawings

  Fig. 1 is a perspective view of a computer for implementing a simulation method according to the present invention.
  Fig. 2 is a flow chart of the simulation method according to a first embodiment of the present invention.
  Fig. 3 is a diagram showing a filler model in the first embodiment.
  Fig. 4 is a diagram showing a polymer model in the first embodiment.
  Fig. 5 is a flow chart of a condition setting process in the first embodiment.
  Fig. 6 is a diagram for explaining potentials between F-particles and P-particles.
  Fig. 7 is a diagram for explaining a cutoff distance for a F-particle.

Fig. 8 is a schematic perspective view of a virtual space in the first embodiment.

Fig. 9 is a flow chart of a simulation process in the first embodiment.

Fig. 10 is a flow chart of a bonding process in the first embodiment.

Fig. 11 is a flow chart of a selecting process in the first embodiment.

Fig. 12 is a diagram for explaining a process for associating the bonding-target P-particles.

Fig. 13 is a diagram for explaining a process for selecting the associated bonding-target P-particles.

Fig. 14 is a diagram for explaining a process for associating the non-selected bonding-target P-particles.

Fig. 15 is a diagram for explaining a process for selecting the associated non-selected bonding-target P-particles.

Fig. 16 is a diagram for explaining a process for binding the selected bonding-target P-particles with the bonding-target F-particles.

Fig. 17 is a perspective view showing the virtual space and the filler models after the molecular dynamics calculation.

Fig. 18 is a flow chart of a simulation method according to a second embodiment of the present invention.

Fig. 19 is a diagram showing a coupling agent model.

Fig. 20 is a flow chart of a condition setting process in the second embodiment.

Fig. 21 is a diagram for explaining potentials for the C-particles.

Fig. 22 is a perspective view of a virtual space in the second embodiment.

Fig. 23 is a flow chart of a simulation process in the second embodiment.

Fig. 24(a) is a diagram showing a state where the C-particles are not yet bound with the P-particles.

Fig. 24(b) is a diagram showing a state where a P-particle is bound with a C-particle.

Fig. 25 is a flow chart of a bonding process in the second embodiment.

Fig. 26 is a flow chart of a selecting process in the second embodiment.

Fig. 27 is a diagram for explaining a process for associating the bonding-target P-particle(s) and/or bonding-target c-particle(s).

Fig. 28 is a diagram for explaining a process for selecting the bonding-target P-particle(s) and/or the bonding-target c-particle(s).

Fig. 29 is a diagram for explaining a process for associating the non-selected bonding-target P-particle(s) and/or bonding-target C-particle(s).

Fig. 30 is a diagram for explaining a process for selecting the associated bonding-target P-particle(s) and/or bonding-target C-particle(s).

Fig. 31 is a diagram for explaining a process for binding the selected bonding-target P-particle(s) and/or bonding-target C-particle(s) with the bonding-target F-particle.

Fig. 32 is a graph showing radial distribution functions of Embodiments 2 and 3.

Fig. 33 is a graph showing radial distribution functions of Embodiments 2 and 4.

Description of Embodiments

Embodiments of the present invention will now be described in detail in conjunction with accompanying drawings.

The simulation method for a high-polymer material according the present invention (shortly, the simulation method) is a computer-implemented method for estimating the dispersion of filler contained in the high-polymer material. For example, the filler may be carbon black, silica, alumina or the like. For example, the high-polymer material may be rubber, resin, elastomer or the like.

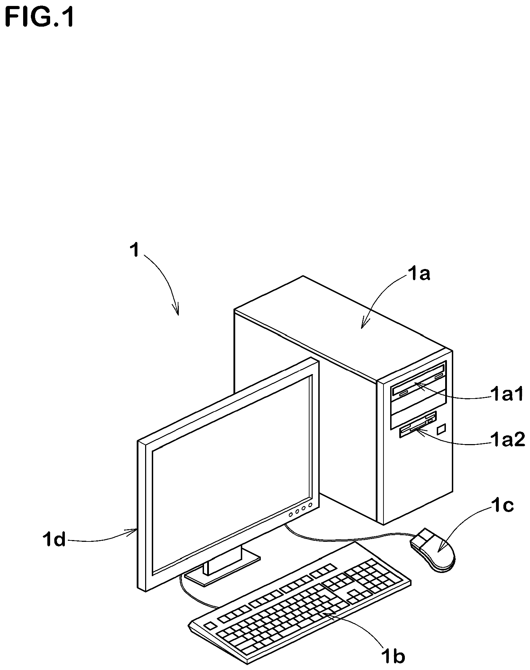

As shown in Fig. 1 for example, the computer 1 for implementing the simulation method according to the present invention comprises a main body 1a, a keyboard 1b, a mouse 1c and a display 1d. The main body 1a comprises an arithmetic processing unit (CPU), memories storage devices such as magnetic disk, disk drives 1a1 and 1a2 and the like. In the storage device, programs/software for carrying out the simulating method are stored.

* Process S1

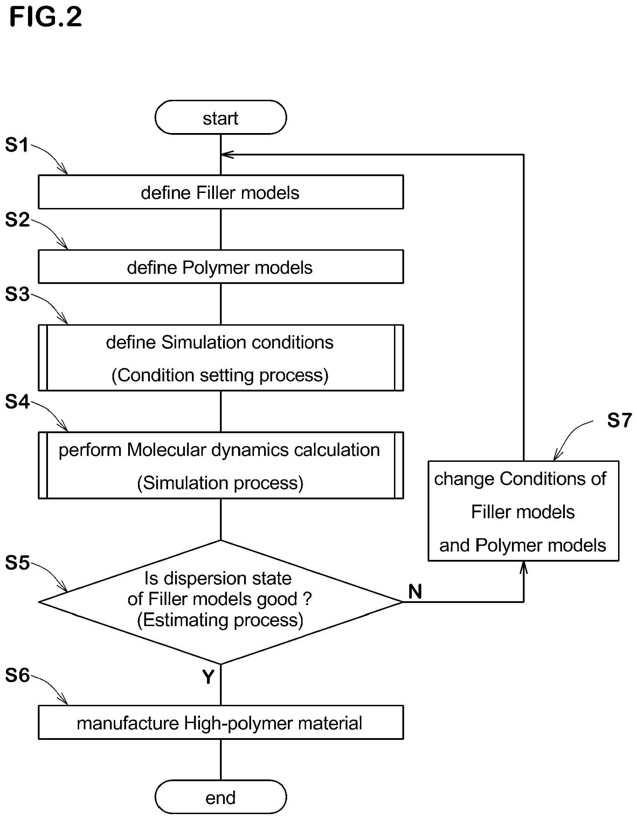

In the simulation method, as shown in Fig. 2, filler models 3 of the filler (particles) are defined. (Process S1)

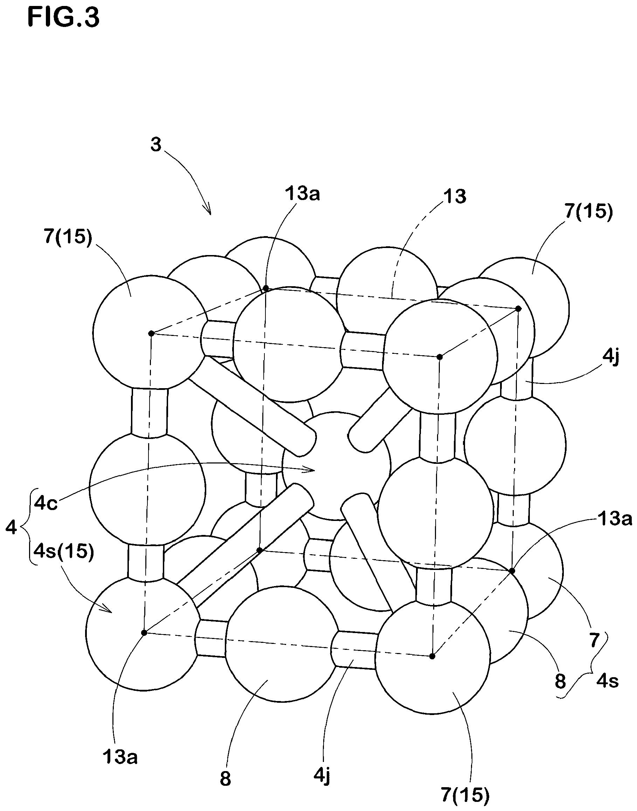

Each filler model 3 is a particle model made up of F-particles 4 as shown in Fig. 3.

The term "F-particle" means a particle of a filler model 3.

The F-particles 4 are arranged to accord with a predetermined polyhedron 13.

In this example, the polyhedron 13 is a regular hexahedron.

More specifically, the F-particles 4 of each filler model 3 are

a single center F-particle 4c disposed at the center of gravity of the polyhedron 13, and
a plurality of surface F-particles 4s disposed at the surface of the polyhedron 13.

The surface F-particles 4s in this example are

apex F-particles 7 disposed at the apexes 13a of the polyhedron 13, and
middle F-particles 8 disposed between the apex F-particles 7.

The center F-particle 4c and the surface F-particles 4s are each defined as a sphere having a certain diameter.
Between the center F-particle 4c and each apex F-particle 7 (surface F-particle 4s), and between each apex F-particle 7 (surface F-particle 4s) and each of the adjacent middle F-particles 8, links 4j having respective equilibrium lengths are defined.
Incidentally, the filler models 3 are numerical data processable with the computer 1 and stored in the computer 1. Such numerical data may include the mass, volume and diameter of each F-particle 4, and initial coordinate values of the F-particles 4.
The equilibrium lengths are equal to the bond distances between the center F-particle 4c and each surface F-particles 4s and between the surface F-particles 4s and 4s, at which the relative positions of the surface F-particles 4s on the polyhedron 13 are stably maintained.
Thereby, the arrangement of the center F-particle 4c and surface F-particles 4s of each filler model 3 can be stably maintained in the form of the polyhedron 13.

* Process S2

In the simulation method, as shown in Fig. 2, the high-polymer material is numerically modeled by polymer models 5.

(Process S2)

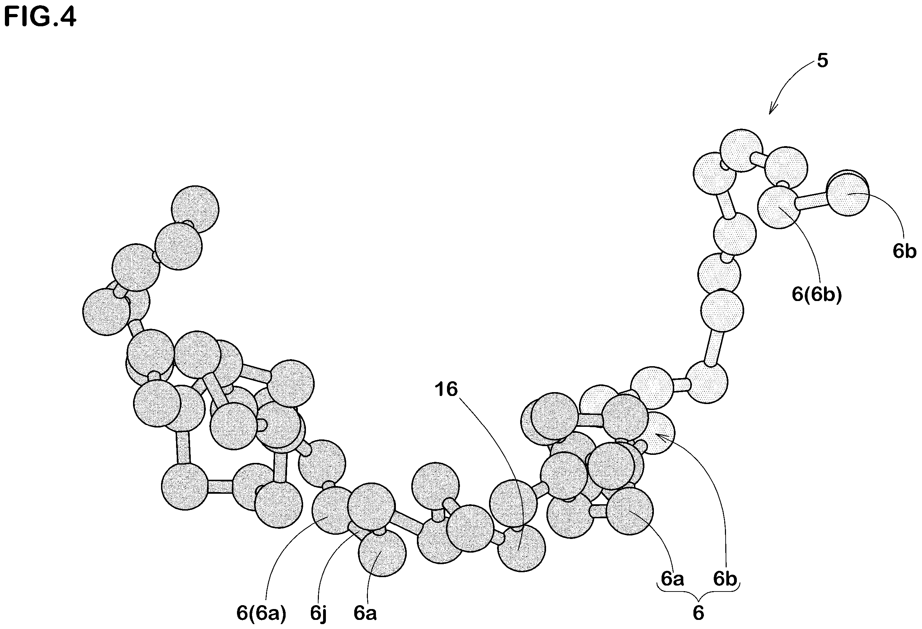

As shown in Fig. 4, each polymer model 5 is a particle model made up of P-particles 6.
The term "P-particle" means a particle of a polymer model 5.
The P-particles 6 are each defined as a sphere having a certain diameter.
In this example, the P-particles 6 include a native P-particle 6a and a denatured P-particle 6b for which different potentials are defined.
Between the native P-particles 6a and 6a, and between the native P-particle 6a and the denatured P-particle 6b, links 6j having respective equilibrium lengths are defined so that the polymer model 5 has a three-dimensional straight-chain structure.
incidentally, the polymer model 5 is numerical data processable with the computer 1 and stored in the computer 1. The numerical data may include the mass, volume, and diameter of each P-particle 6, and initial coordinate values of the P-particles 6.

* Condition setting process S3

In the simulation method, simulation conditions are defined. (Condition setting process S3)

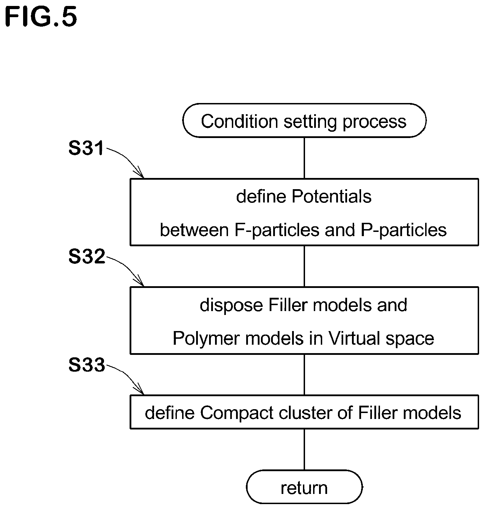

Fig. 5 shows a flow chart of the condition setting process S3.

* Process S31

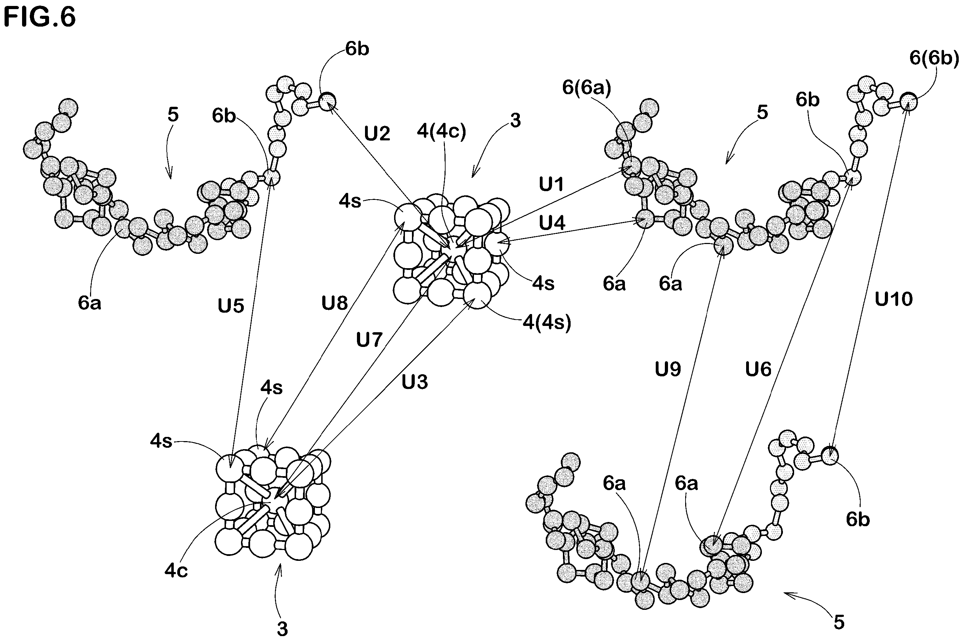

In the condition setting process S3, potentials U are defined between the F-particles (4c and 4s) and the P-particles (6a and 6b) as shown in Fig. 6. (Process S31)
The potential U is a function of the interparticle distance between the concerned two particles and used to calculate a force exerted between the concerned two particles.
If the interparticle distance $r_{ij}$ is less than a predetermined cutoff distance $r_c$, the potential U is defined by the following expression (1):

$$U = \frac{1}{2} a_{ij} \left( 1 - \frac{r_{ij}}{r_c} \right)^2 \qquad \text{---(1)}$$

wherein

$r_{ij}$ is the interparticle distance between the centers of the two particles concerned,
$r_c$ is the cutoff distance between the centers of the two particles concerned, and
$a_{ij}$ is a coefficient for the intensity of the potential defined between the two particles concerned.

Thus, the potential U exerts an interaction force (in this embodiment, a repulsive force).

If the interparticle distance $r_{ij}$ is equal to or more than the predetermined cutoff distance $r_c$, the potential U is defined as zero, and does not exert a force (repulsive force) between the two particles.

In the first embodiment, the following potentials U1-U10 are defined between different models (3 or 5) and (3 or 5).

U1: between center F-particle 4c and native P-particle 6a
U2: between center F-particle 4c and denatured P-particle 6b
U3: between center F-particle 4c and surface F-particles 4s
U4: between surface F-particles 4s and native P-particle 6a
U5: between surface F-particles 4s and denatured P-particle 6b
U6: between native P-particle 6a and denatured P-particle 6b
U7: between center F-particle 4c and center F-particle 4c
U8: between surface F-particles 4s and surface F-particles 4s
U9: between native P-particle 6a and native P-particle 6a
U10: between denatured P-particle 6b and denatured P-particle 6b

In the first embodiment, the values of the coefficient $a_{ij}$ (for example, 25) for the potential U2 between the denatured P-particle 6b and the center F-particle 4c, and the potential U5 between the denatured P-particle 6b and the surface F-particles 4s are set to be less than the values of the coefficient $a_{ij}$ (for example 72) for the potential U1 between the native P-particle 6a and the center F-particle 4c, and the potential U4 between the native P-particle 6a and the surface F-particles 4s.

More specifically, the values of the coefficient $a_{ij}$ for the potentials U1-U10 are set as follows.

potential U1: $a_{ij} = 72$
potential U2: $a_{ij} = 25$
potential U3: $a_{ij} = 50$
potential U4: $a_{ij} = 72$
potential U5: $a_{ij} = 25$
potential U6: $a_{ij} = 72$
potential U7: $a_{ij} = 50$
potential U8: $a_{ij} = 50$
potential U9: $a_{ij} = 50$
potential U10: $a_{ij} = 50$

These values were determined by reference to treatise - J. Chem Phys. 107(11) 4423-4435 (1997) and treatise - Macromolcule vol .39 6744(2006).

Thereby, in comparison with the native P-particle 6a, the repulsive force exerted on the denatured P-particle 6b becomes small. Thus, the denatured P-particle 6b is defined as having a higher affinity for the F-particle (4c, 4s) than the native P-particle 6a. Therefore, the denatured P-particle 6b can model a denatured agent added in the actual high-polymer material. Accordingly, similarly to denaturalizing of the polymer in the actual high-polymer material, by incorporating the denatured P-particle 6b in the polymer model 5, it is possible to change the dispersion of the filler models 3 in the polymer models 5. Thus, the flexibility of the simulation method is increased.

The cutoff distances $r_c$ for the potentials U1-U10 are set as follows.

potential U1: $r_c = 3$
potential U2: $r_c = 3$
potential U3: $r_c = 3$
potential U4: $r_c = 1$
potential U5: $r_c = 1$
potential U6: $r_c = 1$
potential U7: $r_c = 5$
potential U8: $r_c = 1$

potential U9: $r_c = 1$
potential U10: $r_c = 1$

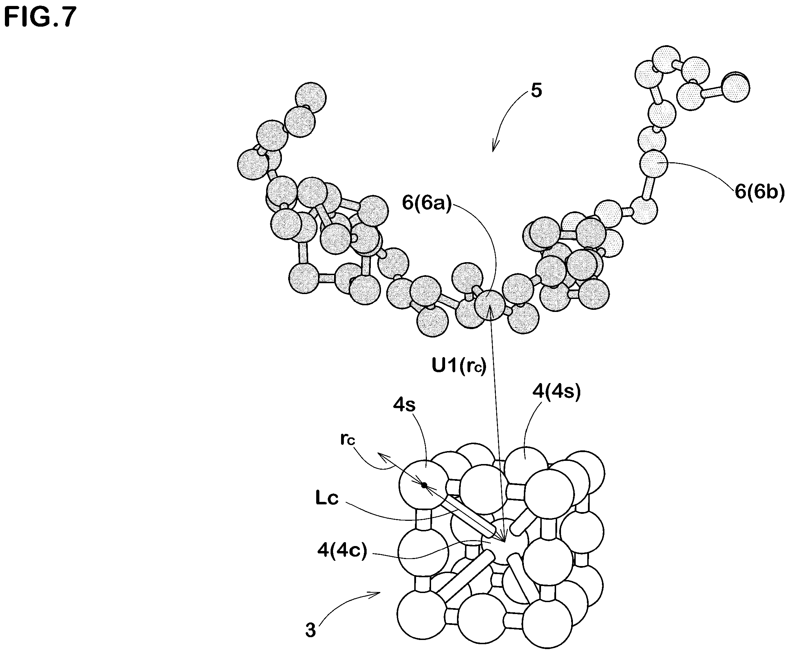

Fig. 7 is a diagram for explaining the cutoff distance for the F-particle.

Each of the cutoff distances $r_c$ for the potentials (for example, potential U1) relating to the center F-particle 4c of a filler model 3 is set to be larger than the cutoff distances $r_c$ for the potentials (for example, potential U4) relating to the surface F-particles 4s of the filler model 3.

In the first embodiment, further, each of the cutoff distances $r_c$ for the potentials (for example, U1) relating to the center F-particle 4c is set to be larger than the sum ($r_c + L_c$) of each of the cutoff distances $r_c$ for the potentials (for example, U4) relating to each surface F-particle 4s and the interparticle distance Lc between the above-mentioned surface F-particle 4s and the center F-particle 4c.

Therefore, by defining the cutoff distance $r_c$ as above in a filler model 3, the potentials (for example, U1-U3 and U7) relating to the center F-particle 4c can exert interaction forces prior to the potentials (for example, U4, U5 and U8) relating to the surface F-particles 4s.

In addition, as the center F-particle 4c is defined as a sphere having a certain diameter, the potentials (U1-U3 and U7) exert radially or in all directions from the center F-particle 4c. Therefore, in a molecular dynamics calculation performed by the computer 1, a filler model 3 is treated as being a sphere approximate to an actual shape of a filler particle, and as a result, the simulation accuracy is improved.

The computer 1 can perform the molecular dynamics calculation for the filler models 3 by the use of substantively only the potentials (for example, U1-U3 and U7) relating to the center F-particle 4c of each filler model 3, excluding particles entered into the cutoff distances $r_c$ of the surface F-particles 4s thereof.

Therefore, in the simulation method according to the present invention, the computational efficiency can be improved. incidentally, numerical data of such potentials U are stored in the computer 1.

* Process S32

Next, with the computer 1, the filler models 3 and the polymer models 5 are randomly disposed (or so defined) within a virtual space 9 having a predetermined volume. (Process S32)

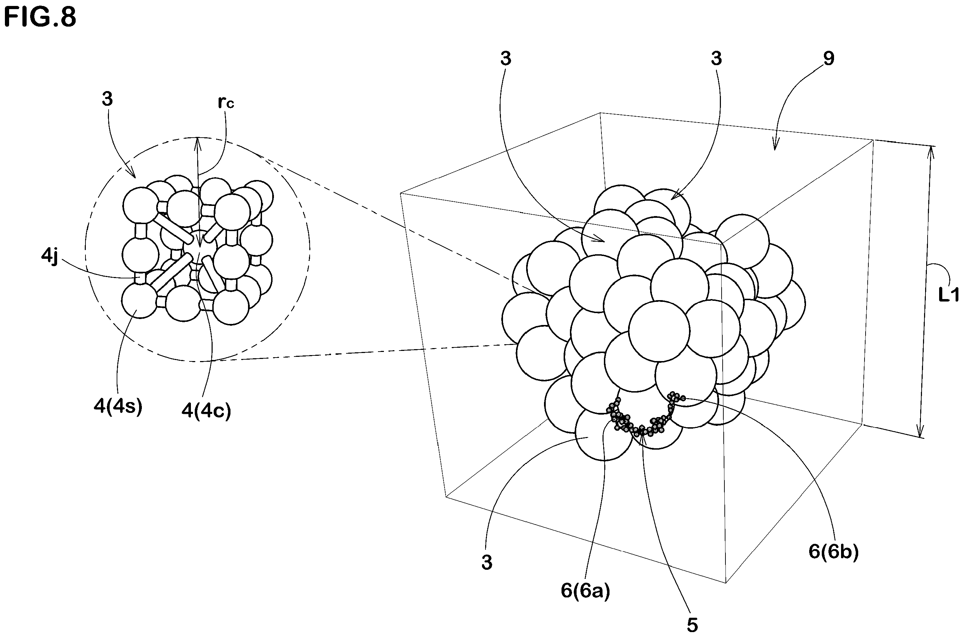

As shown in Fig. 8, the virtual space 9 corresponds to a minute fraction having a microstructure of the rubber polymer as the analysis object.

In this example, the virtual space 9 is defined as being a regular hexahedron whose each side has a length L1 of 30[ σ ] for example.

For example, 100 filler models 3 and 2000 polymer models 5 are randomly disposed in the virtual space 9.

* Process S33

Next, in order to numerically model a compact cluster of filler particles which is liable to occur in the rubber polymer as the analysis object, a compact cluster of the filler models 3 is defined. (Process S33)

In this process S33, the compact cluster of at least two filler models 3 is formed by placing the filler models 3 so that the above-mentioned interaction force between the particles occurs between adjacent filler models 3, in other words, the interparticle distance becomes less than the cutoff distance $r_c$,

* Simulation process S4

Next, as shown in Fig. 2, using the filler models 3 and the polymer models 5 disposed in the virtual space 9, the molecular dynamics calculation is performed by the computer 1.

(Simulation process S4)

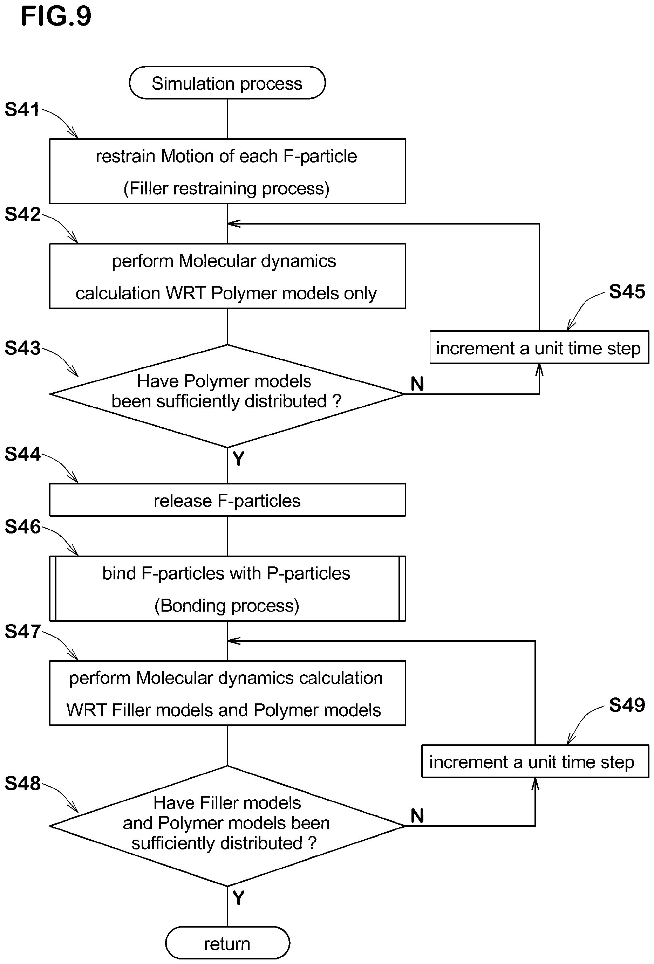

Fig. 9 shows a flow chart of the simulation process S4 in the first embodiment.

* Filler restraining process S41

In the simulation process S4, firstly, as shown in Fig. 8 and Fig. 9, the motion of each of the F-particles 4c and 4s is restrained. (Filler restraining process S41)

In the filler restraining process S41, with the computer 1, the coordinates of the F-particles 4 in the virtual space 9 are fixed so that the F-particles 4 are not moved.

* Process S42

Then, targeting only the polymer models 5, the molecular dynamics calculation is performed by the computer 1.

(Process S42)

In the molecular dynamics calculation, the Newton's equation of motion is applied to all of the polymer models 5 excluding the immovable filler models 3 on the assumption that they accord with the classical dynamics for a specified period of time in the virtual space 9.

And, the P-particles 6a and 6b are traced to obtain their coordinates at each clock time.

During the molecular dynamics calculation, conditions such as the number of each of the F-particles 4c and 4s and P-particles 6a and 6b and the volume and temperature of the virtual space 9, are kept unchanged.

Thus, in the process S42 in the first embodiment, only the polymer models 5 are distributed in the virtual space 9, while preserving the compact cluster of the filler models 3, and a stable arrangement of the polymer models 5 can be obtained.

* Process S43

Next, the computer 1 checks whether or not the polymer models 5 have been sufficiently distributed. (Process S43)

If sufficiently distributed ("Y" in the process S43), then the next process S44 is performed.

If not yet sufficiently distributed ("N" in the process S43), then a unit time step is incremented (Process S45) and the process S42 is repeated.

In order to obtain the stable arrangement of the polymer models 5 through the process S42, the number of steps of the molecular dynamics calculation is preferably not less than 100, more preferably not less than 1000 but preferably not more than 1000000 if the time interval of one step is $0.05[\tau]$ ($[\tau]$ is a unit of time). If less than 100 steps, it becomes difficult to sufficiently distribute the polymer models 5.

Therefore, the polymer models 5 are sufficiently or uniformly distributed through the simulation process S4.

* Process S44

Next, all of the restrained F-particles 4 of the filler models 3 are released in order to allow the filler models 3 to move within the virtual space 9 in the after-mentioned process S47. (Process S44)

In this process S44, the computer 1 unfixes the coordinates of the F-particles 4.

* Bonding process S46

Next, the F-particles 4 and the P-particles 6 are bound.

(Bonding process S46)

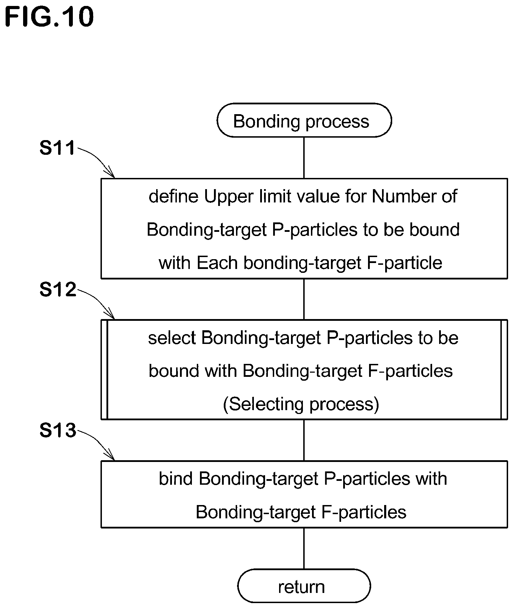

Fig. 10 is a flow chart of the bonding process S46.

In the bonding process S46, bonding-target F-particles 15 are selected from the F-particles 4, and bonding-target P-particles 16 are selected from the P-particles 6.

The bonding-target F-particles 15 are bound with the bonding-target P-particles 16 via links to simulate chemical bond.

In this example, the bonding-target F-particles 15 of each filler model 3 are eight F-particles 4 which are the apex F-particles 7 positioned at the apexes 13a of the polyhedron 13 as shown in Fig. 3.

The bonding-target P-particle 16 of each polymer model 5 is, as shown in Fig. 4, one positioned at the almost center in the length of the straight chain arrangement of the P-particles 6.

* Process S11

In the bonding process S46, an upper limit value Su for the number Sa of the bonding-target P-particles 16 to be bound with each bonding-target F-particle 15 is defined.

(Process S11)

The upper limit value Su is determined based on the total number Nb of the bonding-target F-particles 15 existing in the virtual space 9 and the total number Na of the bonding-target P-particles 16 existing in the virtual space 9.

According to the present invention, the upper limit value Su is defined to satisfy the following conditional expression (2):

$$Na/Nb-1 =< Su =< Na/Nb+1 \quad --(2).$$

In this example, each polymer model 5 has one bonding-target P-particle 16, therefore, the total number Na is equal to the total number (for example, 2000) of the polymer models 5 existing in the virtual space 9.

Each filler model 3 has eight bonding-target F-particles 15, therefore, the total number Nb is equal to the total number (for example 100) of the filler models 3 existing in the virtual space 9 multiplied by eight.

The Na/Nb means the number Sa when the bonding-target P-particles 16 are evenly bound with the bonding-target F-particles 15.

If Na = 2000 and Nb = 800, Na/Nb = 2.5 (2.5-1 =< Su =< 2.5 + 1).

Since the Su is a positive integer, the upper limit value Su for each bonding-target F-particle 15 is defined as 2 or 3.

* Selecting process S12

In the bonding process S46, next, the bonding-target P-particles 16 to be bound with the bonding-target F-particles 15 are selected by the computer 1. (Selecting process S12)

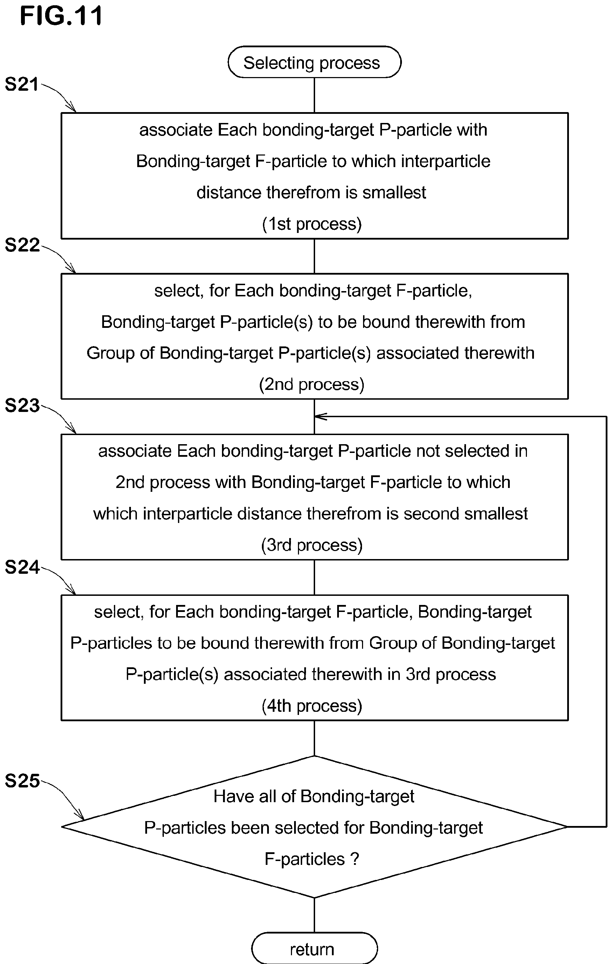

Fig. 11 is a flow chart of the selecting process S12.

* 1st process S21

In the selecting process S12, firstly, each bonding-target P-particle 16 is associated with a bonding-target F-particle 15 to which interparticle distance Lp therefrom is smallest. (1st process S21)

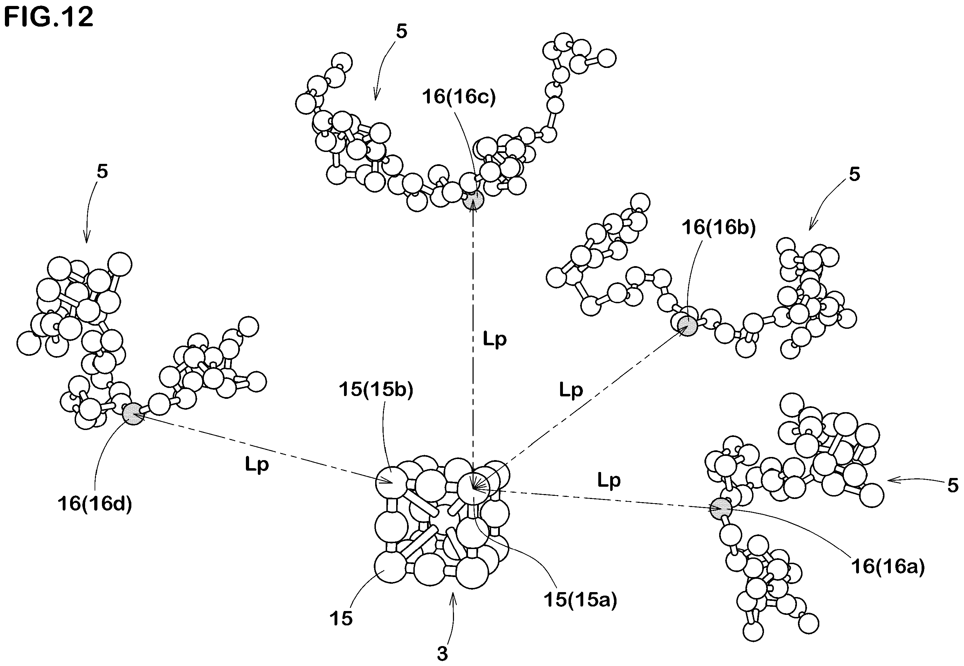

Fig. 12 is a diagram for explaining this process S21.

In the 1st process S21, for each bonding-target P-particle 16 in the virtual space 9, a bonding-target F-particle 15 to which interparticle distance Lp from the concerned bonding-target P-particle 16 is smallest, is searched.

Then, the searched-out bonding-target F-particle 15 is associated with the concerned bonding-target P-particle 16. Here, the interparticle distance Lp is the distance between centers of the concerned particles 15 and 16.

Data about such associations, for example, identification numbers of each bonding-target P-particle 16 and the bonding-target F-particle 15 to be associated therewith, and their coordinate values in relation to the virtual space 9, are stored in the computer 1.

In an example shown in Fig. 12, two bonding-target F-particles 15 are shown, which are a first bonding-target F-particle 15a and a second bonding-target F-particle 15b.

with respect to the first bonding-target F-particle 15a, three bonding-target P-particles 16 are associated therewith, which are a first bonding-target P-particle 16a, a second bonding-target P-particle 16b and a third bonding-target P-particle 16c in ascending order of the interparticle distance Lp from the concerned bonding-target F-particle 15.

with respect to the second bonding-target F-particle 15b, a single bonding-target P-particle 16 is associated therewith, which is a fourth bonding-target P-particle 16d.

In Fig. 12, each chain double-dashed line indicates an association of a bonding-target F-particle 15 with a bonding-target P-particle 16.

* 2nd process S22

In the selecting process S12, next, for each bonding-target F-particle 15, one or more bonding-target P-particles 16 to be bound therewith are selected from a group of the bonding-target P-particle(s) 16 associated therewith (hereinafter, the "bonding-target P-particle group"). (2nd process S22)

The selection of the bonding-target P-particle(s) 16 is made in the ascending order of the interparticle distance Lp from the concerned bonding-target F-particle 15 so that the number of the selected bonding-target P-particle(s) 16 does not exceed the above-mentioned upper limit value Su for the number Sa.

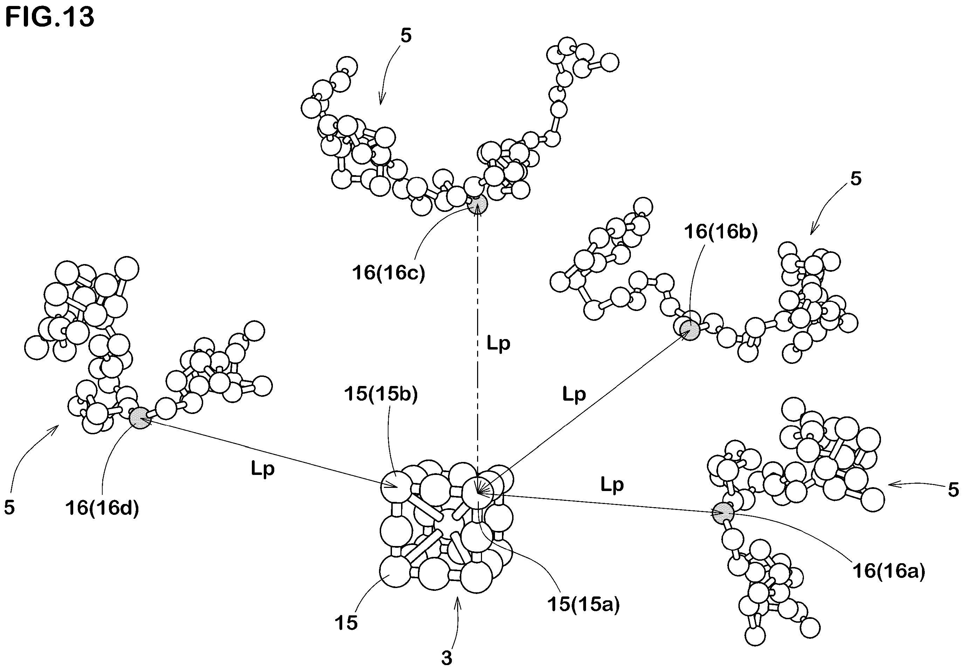

Fig. 13 is a diagram for explaining a process for selecting the bonding-target P-particle(s) 16, where the upper limit value Su is 2.

As explained above in connection with Fig. 12, associated with the first bonding-target F-particle 15a are three bonding-target P-particles 16, the number of which is more than the upper limit value Su (=2).

Therefore, from the group of the bonding-target P-particles (16a, 16b and 16c) associated with the first bonding-target F-particle 15a, a number (which is, in this example, equal to the upper limit value Su) of the bonding-target P-particles (16a and 16b) are selected in their ascending order of the interparticle distance Lp.

Then, the selected bonding-target P-particles (16a and 16b) are defined as particles to be bound with the first bonding-target F-particle 15a as shown in Fig. 13, wherein the selected association of a bonding-target F-particle 15 with a bonding-target P-particle 16 is indicate by a solid line instead of the above-mentioned chain double-dashed line.

On the other hand, each bonding-target P-particle not selected from the group (for example, the third bonding-target P-particle 16c to which interparticle distance Lp is largest) is defined as a particle not to be bound with the first bonding-target F-particle 15a.

In the case of the second bonding-target F-particle 15b, only one bonding-target P-particle 16 (fourth bonding-target P-particle 16d) is associated therewith, and
the number of which is less than the upper limit value Su (=2). Therefore, the only one P-particle 16d is defined as a particle to be bound with the second bonding-target F-particle 15b.

In the 2nd process S22, as described above, the bonding-target P-particles 16 are selected so that the number Sa of the bonding-target P-particle(s) 16 to be bound with each bonding-target F-particle 15 does not exceed the upper limit value Su.

Data about such selections, for example, identification numbers of each bonding-target F-particle 15 and the bonding-target P-particle(s) 16 selected therefor and their coordinate values in relation to the virtual space 9, are stored in the computer 1.

* Third process S23

In the selecting process S12, next, each bonding-target P-particle 16 not selected in the 2nd process S22 is associated with another bonding-target F-particle 15. (Third process S23)

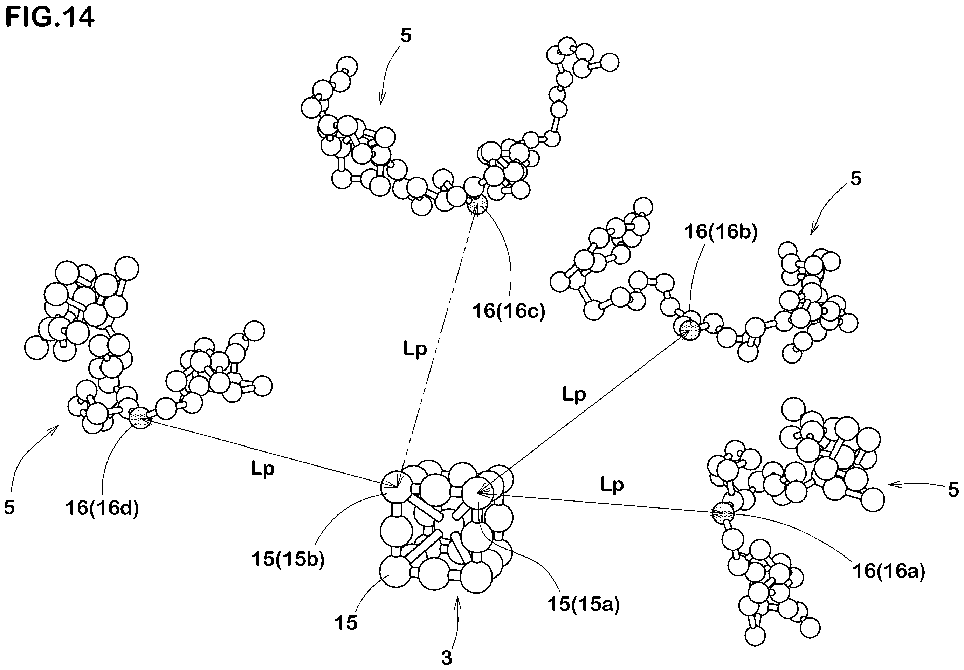

Fig. 14 is a diagram for explaining this process S23.

in the third process S23, firstly, for each bonding-target P-particle 16 not selected in the 2nd process S22, there is searched a bonding-target F-particle 15 to which interparticle distance Lp from the concerned not-selected bonding-target P-particle 16 is second smallest next to the smallest interparticle distance Lp of the bonding-target F-particle 15 associated, in the 1st process S21, with the concerned not-selected bonding-target P-particle 16, and the searched-out bonding-target F-particle 15 is associated with the concerned bonding-target P-particle 16.

Data about such association are stored in the computer 1.

In an example shown in Fig. 14, the third bonding-target P-particle 16c is a bonding-target P-particle 16 not selected in the 2nd process S22.

In the third process S23, there is searched a bonding-target F-particle 15 to which interparticle distance Lp from the third bonding-target P-particle 16c is second smallest next to the smallest interparticle distance Lp from the third bonding-target P-particle 16c to the first bonding-target F-particle 15a.

In this example, the second bonding-target F-particle 15b is searched out.

Then, the second bonding-target F-particle 15b is associated with the third bonding-target P-particle 16c.

* Fourth process S24

in the selecting process S12, next, for each bonding-target F-particle 15, bonding-target P-particles 16 to be bound therewith are selected from a group of the bonding-target P-particle(s) associated with the concerned bonding-target F-particle 15 in the third process S23. (Fourth process S24)

In the same way as in the 2nd process S22, the selection of the bonding-target P-particle(s) 16 is made in the ascending order of the interparticle distance Lp so that the number of the selected bonding-target P-particle(s) 16 satisfies the above-mentioned upper limit value Su for the number Sa.

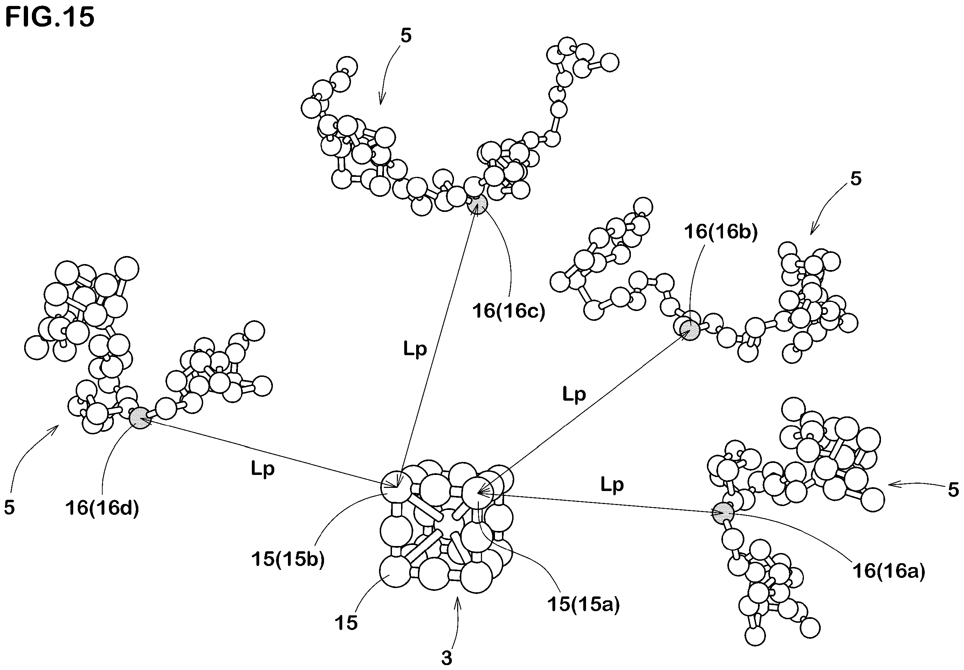

Fig. 15 is a diagram for explaining the process S24.

In this example, for the second bonding-target F-particle 15b, the single bonding-target P-particle 16 (fourth bonding-target P-particle 16d) has been already selected as explained above.

Therefore, in order that the number Sa not exceed the upper limit value Su (=2), only the third bonding-target P-particle 16c is associated with the second bonding-target F-particle 15b. And the third bonding-target P-particle 16c is defined as a particle to be bound with the second bonding-target F-particle 15b.

If more than two bonding-target P-particles 16 are associated with the second bonding-target F-particle 15b, the selection from the group of the more than two bonding-target P-particles 16 is made in the ascending order of the interparticle distance Lp so that the resultant number Sa does not exceed the upper limit value Su.

Thus, each bonding-target P-particle 16 (not shown) to which interparticle distance Lp is more than the second smallest, is defined as a particle not bound with the second bonding-target F-particle 15b.

In the fourth process S24, as described above, the bonding-target P-particle(s) 16 to be bound with each bonding-target F-particle 15 is(are) selected from a group of the bonding-target P-particle(s) 16 associated therewith through the third process S23 so that the resultant number Sa does not exceed the upper limit value Su.

Data about such selections are stored in the computer 1.

* Process S25

In the selecting process S12, next, it is checked whether or not all of the bonding-target P-particles 16 have been selected for the bonding-target F-particles 15.

(Process S25)

If all of the bonding-target P-particles 16 have been selected, then the next process S13 is performed.

If not yet selected, then with respect to each bonding-target P-particle 16 not selected, the third process S23 and the fourth process S24 are repeated.

Therefore, through the selecting process S12, all of the bonding-target P-particles 16 are selected for the bonding-target F-particles 15.

But, in this embodiment, there is a possibility of existence of a bonding-target F-particle 15 for which no bonding-target P-particle 16 is selected finally.

* Process S13

In the bonding process S46, next, the bonding-target P-particles 16 selected through the selecting process S12 are bound with the bonding-target F-particles 15 via links 10 in order that the filler models 3 and the polymer models 5 can simulate chemical bond between the filler and the polymer.

(Process S13)

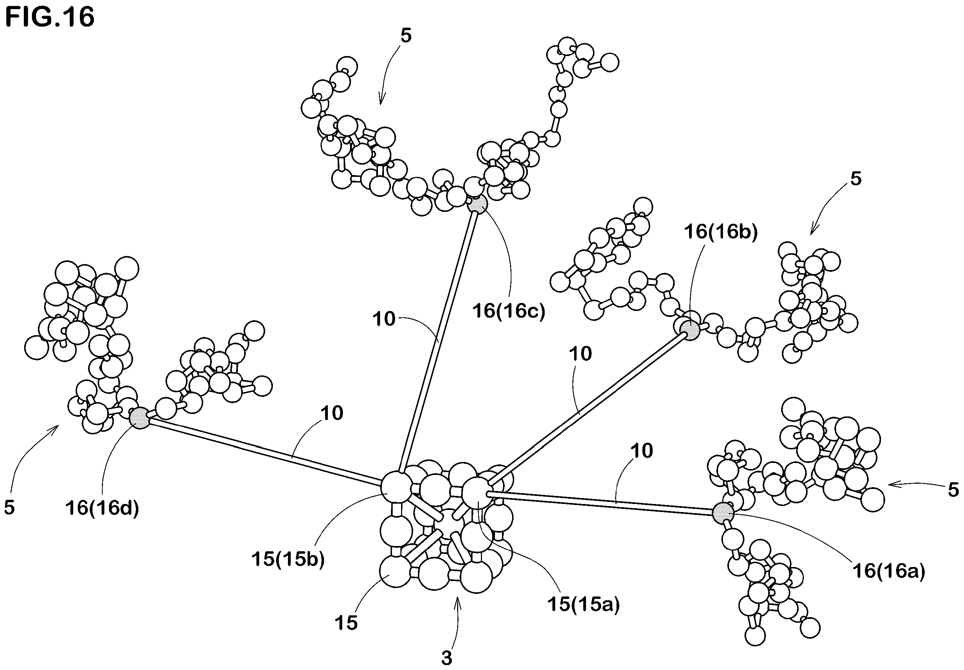

Fig. 16 is a diagram for explaining this process S13.

* Process S47

In the simulation process S4, next, with respect to the filler models 3 and the polymer models 5, a molecular dynamics calculation is performed. (Process S47)

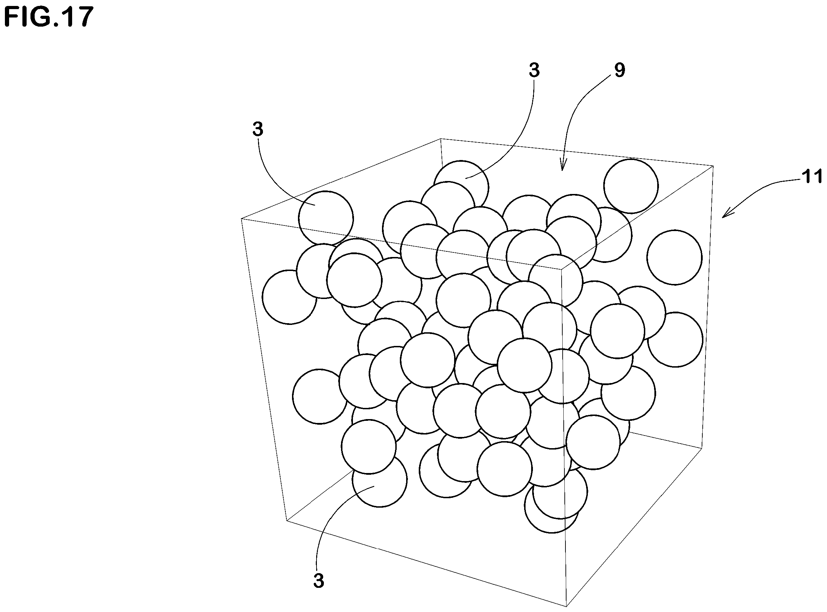

Fig. 17 is a conceptual perspective view of a simulation model after the molecular dynamics calculation, wherein the polymer models 5 are omitted.

In the molecular dynamics calculation performed in the process S47, the Newton's equation of motion is applied similarly to the process 542.

And, the F-particles 4c and 4s of each filler model 4 (Fig. 3) and the P-particles 6a and 6b of each polymer model 5 (Fig. 4) are traced to obtain their coordinates at each clock time. During the molecular dynamics calculation, the conditions, such as the number of each of the F-particles 4c and 4s and P-particles 6a and 6b and the volume and temperature of the virtual space 9, are kept unchanged.

Through the process S47, as shown in Fig. 17, the filler models 3 and the polymer models 5 (not shown) are distributed in the virtual space 9.

In the bonding process S46, as explained, the number Sa of the bonding-target P-particle(s) 16 to be bound with each bonding-target F-particle 15 is limited to a value not more than the predetermined upper limit value Su, so that the summation of the forces of the bonding-target P-particles 16 exerted on each bonding-target F-particle 15 does not increase excessively during the molecular dynamics calculation, therefore, a stable molecular dynamics calculation without failure is possible.

In this embodiment, the upper limit value su is set so as to satisfy the above-mentioned conditional expression (2), based on the number Sa of the bonding-target P-particles 16 per a bonding-target F-particle 15 when the bonding-target P-particles 16 are evenly bound with the bonding-target F-particles 15. Consequently, the bonding-target P-particles 16 can be evenly bound with the bonding-target F-particles 15, and it is possible to prevent an excessive increase in the summation of the forces of the bonding-target P-particles 16 exerted on each bonding-target F-particle 15.

In this embodiment, since the bonding-target F-particles 15 are only the apex F-particles 7 disposed at the apexes 13a of the polyhedron 13, it is possible to provide a space between the bonding-target P-particle(s) 16 bound with a bonding-target F-particle 15 and the bonding-target P-particle(s) 16 bound with an adjacent bonding-target F-particle 15.

Thereby, it is possible to prevent the interaction between the bonding-target P-particles 16 from becoming excessively increased, and it is possible to stably perform the molecular dynamics calculation.

\* Process S48

Next, the computer 1 judges whether or not the filler models 3 and the polymer models 5 have been sufficiently distributed. (Process S48)

If sufficiently distributed ("Y" in the process S48), then the next estimating process S5 (Fig. 2) is performed.

If not yet sufficiently distributed ("N" in the process 548), then a unit time step is incremented (Process 549), and the process S47 is repeated.

In the simulation process S4, therefore, a structurally-relaxed high-polymer material model 11 in which the filler models 3 and the polymer models 5 are fully distributed, can be defined.

In order to effectually distribute the filler models 3 and the polymer models 5, the number of steps of the molecular dynamics calculation performed in the process S47, is preferably not less than 1000, more preferably not less than 5000 but preferably not more than 1000000 if the time interval of one step is 0.05[ $\tau$ ].

If the number of steps is less than 1000, there is a possibility that the filler models 3 and the polymer models 5 are not sufficiently distributed.

\* Estimating process S5

As shown in Fig. 2, in the simulation method according to the first embodiment, next, it is judged whether the dispersion state of the filler models 3 is good or not.

(Estimating process S5)

In this process S5, similarly to the patent document 1, with respect to only the center F-particle 4c of each filler model 3, the radial distribution function is computed.

The radial distribution function is a function which expresses a probability density that another center F-particle 4c can exist at a distance r from a certain center F-particle 4c (Fig. 3). Here, the distance r is that between the centers of the center F-particles 4c.

In the first embodiment, the dispersion state of the filler models 3 is obtained by computing the radial distribution functions with respect to only the center F-particles 4c. Therefore, it is possible to control an increase in the computational cost.

More specifically, in this estimating process S5, with the computer 1, it is checked whether the results of the radial distribution functions are within the predetermined permissible limits or not.

If within the permissible limits, namely, the dispersion state is good, then according to the high-polymer material model 11, an actual high-polymer material is manufactured. (Process s6)

If outside the permissible limits, namely, the dispersion state is not good, then the conditions previously set to the filler models 3 and the polymer models 5 are changed, for example, taking the results of the radial distribution functions into account (process S7), and then the processes S1to S5 are repeated.

Thereby, the structure of the high-polymer material model 11 (shown in Fig. 17) can be effectively relaxed so as to simulate a structure of an actual high-polymer material.

In the simulation method according to the first embodiment, since the structures of the filler models 3 and the polymer models 5 and the potentials can be arbitrarily defined, it is possible to estimate the properties and performances of a nonexistent unknown high-polymer material.

In the simulation method according to the first embodiment, the dispersion of filler contained in a high-polymer material is estimated, using the filler models 3 and the polymer models 5 disposed in the virtual space 9.

But, the high-polymer material is not limited to this type. The high-polymer material can be a high-polymer material further containing a coupling agent for coupling the filler with the polymer as follows.

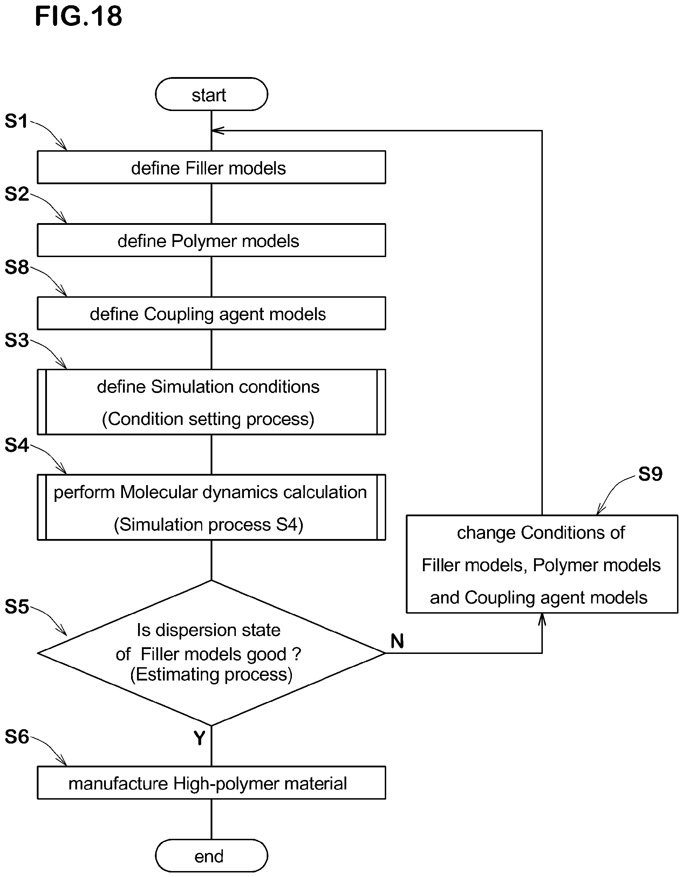

Fig. 18 is a flow chart of the simulation method according to a second embodiment of the present invention.

In the following description, with respect to the processes, models and the like of the second embodiment which are the same as those in the first embodiment, the same reference numbers and characters are assigned and the descriptions are omitted in certain instances.

In the simulation method according to the second embodiment, as described above in connection with the first embodiment, the process S1for defining filler models 3 and the process S2 for defining the polymer models 5 are performed.

* Process S8

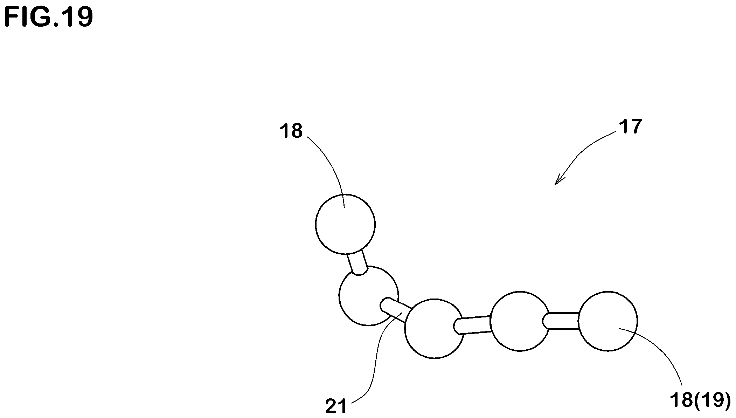

In the second embodiment, coupling agent models 17 of the coupling agent are defined. (Process S8) Fig. 19 is a diagram showing a coupling agent model 17.

The coupling agent model 17 is a particle model made up of at least one, in this example, a plurality of C-particles 18. The term "C-particle" means a particle of a coupling agent model 17.

The C-particles 18 are each defied as a sphere having a certain diameter.

Between the C-particles 18, links 21 having respective equilibrium lengths are defined.

In this example, each coupling agent model 17 has a three-dimensional straight-chain structure.

The number of the C-particles 18 of each coupling agent model 17 is smaller than the number of the P-particle 6 of a polymer model 5 in order to simulate the coupling agent having a molecular weight smaller than that of a molecular chain of the high-polymer material.

incidentally, if a coupling agent model 17 is made up of a single C-particle 18, there is no link 21.

In this example, all of the coupling agent models 17 are made up of the identical number of the C-particles 18.

But, it is also possible to employ plural kinds of coupling agent models 17 made up of different numbers of the C-particles 18, for example, in order to estimate the effect of variations of the lengths of the links of the coupling agent on the dispersion of the filler.

incidentally, the coupling agent models 17 are numerical data processable with the computer 1 including the mass, volume, diameter, initial coordinate values and the like of each C-particle 18 and stored in the computer 1.

* Condition setting process S3

In the simulation method according to the second embodiment, next, simulation conditions are defined.

(Condition setting process S3)

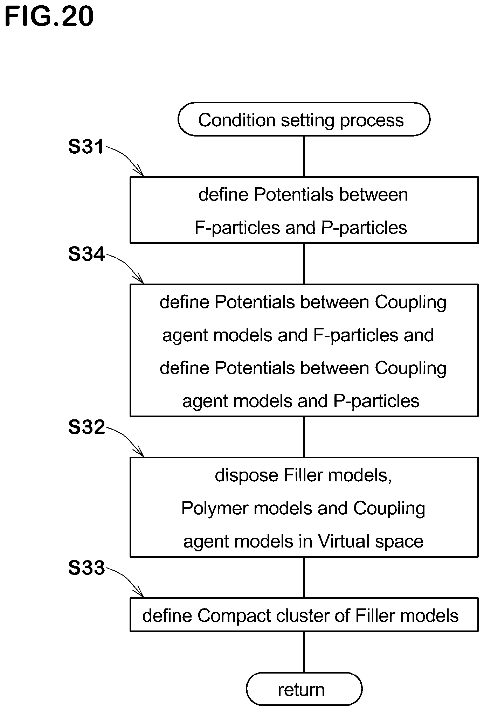

Fig. 20 is a flow chart of the condition setting process S3 according to the second embodiment.

In this process S3, in the same way as in the first embodiment, the above-mentioned process S31 for defining potentials between the F-particles 4c and 4s and the P-particles 6a and 6b is performed.

The potentials U1-U10 between the F-particles 4c and 4s and the P-particles 6a and 6b shown in Fig. 6 are defined in the same way as in the first embodiment.

* Process S34

In the condition setting process S3 in the second embodiment, next, potentials U are defined between the C-particles 18 and the F-particles 4c and 4s, and between the C-particles 18 and the P-particles 6a and 6b.

(Process S34)

The potential U is defined by the above-mentioned expression (1).

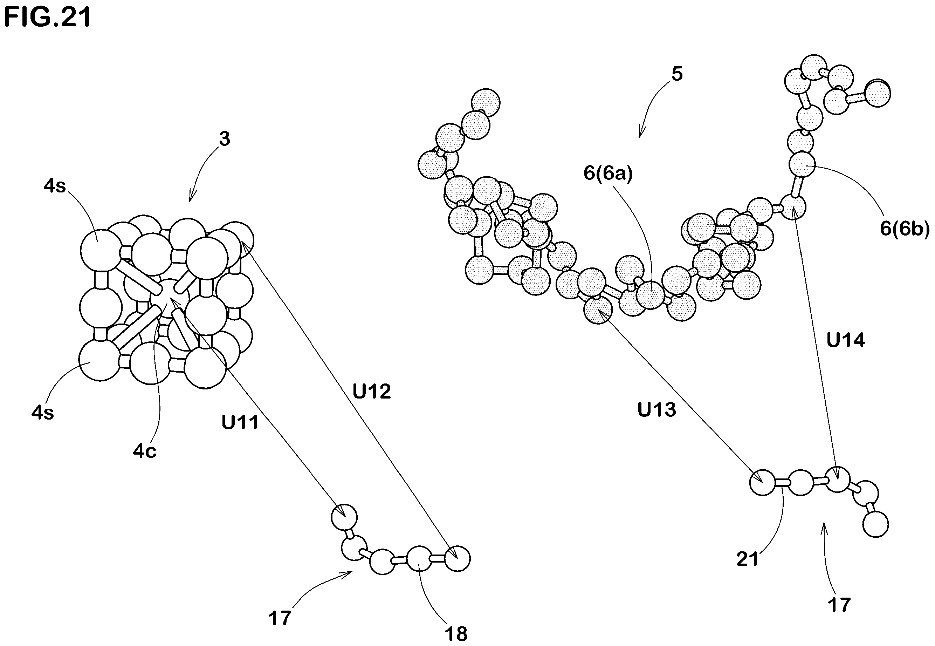

Fig. 21 is a diagram for explaining the potentials relating to the C-particles 18.

As shown, potentials U11 - U14 are defined as follows.

U11: between C-particle 18 and center F-particle 4c
U12: between C-particle 18 and surface F-particles 4s
U13: between C-particle 18 and native P-particle 6a
U14: between C-particle 18 and denatured P-particle 6b

In this example, the coefficient $a_{ij}$ for each potential U11-U14 is defined as follows.

potential U11: $a_{ij} = 50$
potential U12: $a_{ij} = 50$
potential U13: $a_{ij} = 50$
potential U14: $a_{ij} = 50$

The reason for setting the same value to the potentials U11-U14 is to even the repulsive force of the coupling agent model 17 exerted on the filler model 3 and the repulsive force of the coupling agent model 17 exerted on the polymer model 5, namely, to simulate a coupling agent in which the affinity for the filler is the same as the affinity for the polymer.

In the second embodiment, it is also possible that the coefficients $a_{ij}$ for the potentials U11-U14 are set to different values in order to simulate a coupling agent in which the affinity for the filler is different from the affinity for the polymer, for example as follows.

potential U11: $a_{ij} = 10$
potential U12: $a_{ij} = 10$
potential U13: $a_{ij} = 100$
potential U14: $a_{ij} = 100$

In this example, the repulsive force between the filler model 3 and the coupling agent model 17 can be smaller than the repulsive force between the polymer model 5 and the coupling agent model 17, and the affinity of the coupling agent model 17 becomes higher for the filler model 3 than the polymer model.

Further, the cutoff distance $r_c$ in the expression (1) is set as follows.

potential U11: $r_c = 3$
potential U12: $r_c = 1$
potential U13: $r_c = 1$
potential U14: $r_c = 1$

The cutoff distance $r_c$ of the potential U11 relating to the center F-particle 4c of the filler model 3 is set to be larger than the cutoff distance $r_c$ of the potential U12 relating to the surface F-particles 4s of the filler model 3, and the cutoff distance $r_c$ of the potential U11 is set to be larger than the summation $(r_c + Lc)$ of the cutoff distance $r_c$ of the potential U12 and the interparticle distance Lc (see Fig. 7) between the surface F-particles 4s and the center F-particle 4c. Therefore, the filler model 3 can exert the potential U11 (Fig. 21) relating to the center F-particle 4c prior to the potential U12 relating to the surface F-particles 4s.

Further, as the center F-particle 4c is expressed by the sphere having the certain diameter, it is possible to exert the potential U11 radially. Therefore, in the simulation process S4, the filler model 3 can be treated as a sphere approximate to the actual filler, and the simulation accuracy can be improved.

* Process S32

In the condition setting process S3 in the second embodiment, a plurality of the filler models 3, a plurality of polymer models 5 and a plurality of coupling agent models 17 are disposed in the predetermined virtual space 9. (process S32)

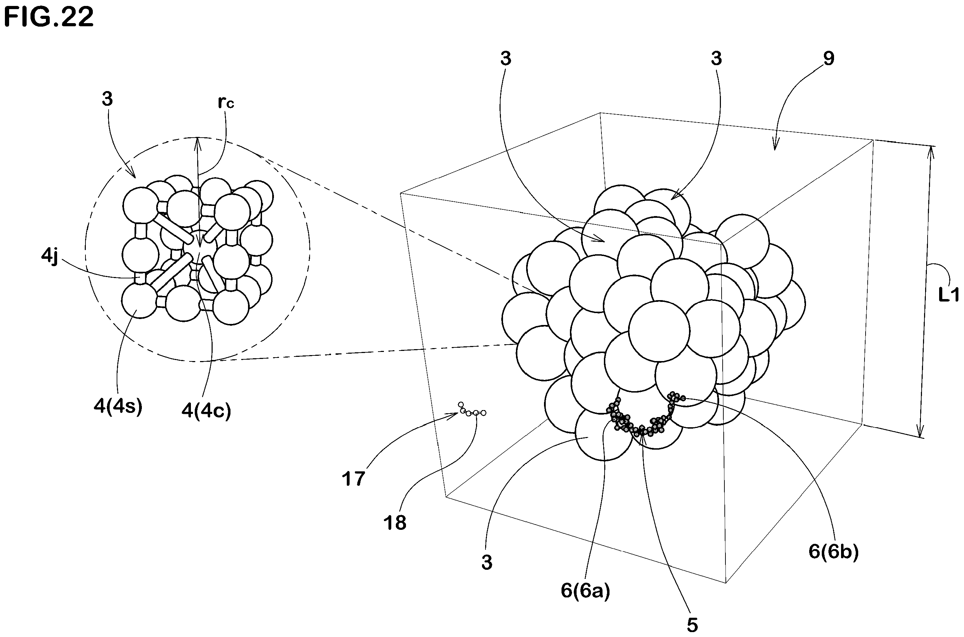

As to the virtual space 9, the virtual space show in Fig. 8 employed in the first embodiment can be employed in the second embodiment as shown in Fig. 22.

For example, in the virtual space 9, 100 filler models 3, 1000 polymer models 5, and 1000 coupling agent models 17 are randomly disposed.

* Process 533

The process S33 for forming a compact cluster of the filler models 3 is performed in the same way as in the first embodiment. (Process S33)

As to the technique for forming a compact cluster of the filler models 3, that employed in the first embodiment is employed.

* Simulation process S4

In the simulation method according to the second embodiment, next, with the computer 1, a molecular dynamics calculation is performed using the filler models 3, the polymer models 5 and the coupling agent models 17 disposed in the virtual space 9. (Simulation process S4)

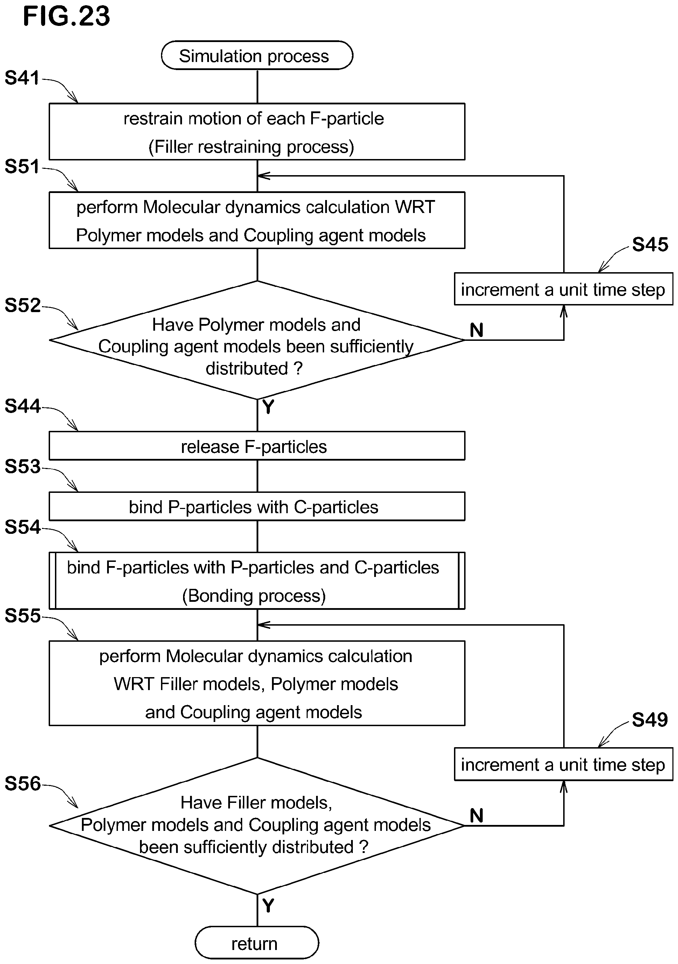

Fig. 23 is a flow chart of the simulation process S4 in the second embodiment.

* Filler restraining process S41

In the simulation process S4 in the second embodiment, first, the filler restraining process S41 for restraining the motion of each of the F-particles 4c and 4s (shown in Fig. 22) is performed in the same way as in the first embodiment.

* Process S51

Then, with respect to the polymer models 5 and the coupling agent models 17, a molecular dynamics calculation is performed. (Process S51)

In the molecular dynamics calculation in this process S51, the Newton's equation of motion is applied to all of the polymer models 5 and the coupling agent models 17 excluding the immovable filler models 3 on the assumption that they accord with the classical dynamics for a specified period of time in the virtual space 9.

And the P-particles 6a and 6b and the C-particles 18 are traced to obtain their coordinates at each clock time.

During the molecular dynamics calculation, the conditions, such as the numbers of the F-particles 4c and 4s, P-particles 6a and 6b and C-particles 18 and the volume and temperature of the virtual space 9 are kept unchanged.

Thus, in the process S51, only the polymer models 5 and the coupling agent models 17 are distributed within the virtual space 9, while keeping the compact cluster of the filler models 3 unchanged, and a stable arrangement of the polymer models 5 and the coupling agent models 17 can be obtained.

* Process S52

In the simulation process S4 in the second embodiment, next, with the computer 1, it is judged whether the polymer models 5 and the coupling agent models 17 are sufficiently distributed or not. (Process S52)

If sufficiently distributed ("Y" in the process S52), then the next process S44 is performed.

If not yet sufficiently distributed ("N" in the process S52), then a unit time step is incremented (Process S45), and the process S51 is repeated.

Therefore, in the simulation process S4 in the second embodiment, the polymer models 5 and the coupling agent models 17 can be effectually distributed.

In order to obtain a stable arrangement of the polymer models 5 and the coupling agent models 17, similarly to the first embodiment, the number of steps of the molecular dynamics calculation is preferably set to not less than 1000 if the time interval of one step is 0.05[ $\tau$ ].

* Process S44

In the process S44, all of the restrained F-particles 4 of the filler models 3 are released in the same way as in the first embodiment.

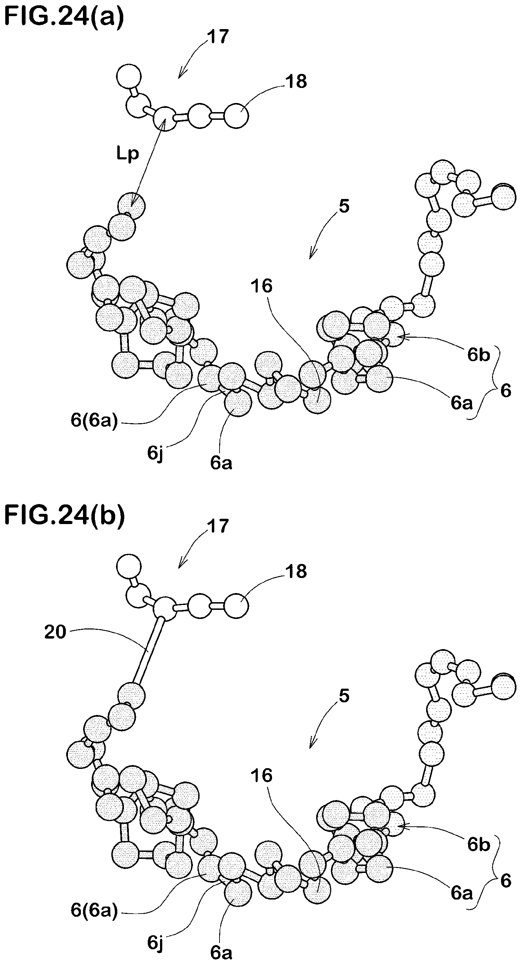

Fig. 24(a) and Fig. 24(b) are diagrams showing the P-particles 6 and the C-particles 18 before bound and after bound, respectively.

* Process S53

In the simulation process S4, next, the P-particles 6 are bound with the C-particles. (Process S53)

In this process S53, a P-particle 6 and a C-particle 18 are bound with each other via a link 20 when the interparticle distance therebetween becomes a predetermined value L3 or less to simulate a chemical bond between the coupling agent and the polymer.

Preferably, the predetermined value L3 is 0 % to 200 % of the cutoff distance $r_C$ (Fig. 7) for the potential relating to the concerned C-particle 18.

If, with respect to one coupling agent model 17, a plurality of P-particles 6 whose interparticle distances are not more than L3 exist, then the coupling agent model 17 is bound with only one P-particle 6 of which interparticle distance is not more than the other(s), which usually has the smallest interparticle distance Lp in order that the forces of a plurality of P-particles 6 are not accumulated on one C-particle 18, and thereby to avoid calculation failure.

* Bonding process S54

In the simulation process S4 in the second embodiment, next, the F-particles 4 are bound with the P-particles 6 and the C-particles to simulate chemical bond between the filler, polymer and coupling agent. (Bonding process S54)

In this process S54, each bonding-target F-particle 15 selected from a plurality of the F-particles 4 is bound with a bonding-target P-particle 16 selected from a plurality of the P-particles 6 via a link and/or bound with a bonding-target C-particle 19 selected from a plurality of the C-particle 18 via a link.

The bonding-target F-particle(s) 15 may be arbitrarily selected from the F-particles 4 of each filler model 3.

As shown in Fig. 3, in the second embodiment, similarly to the first embodiment, the eight F-particles 4 or the apex F-particles 7 are the bonding-target F-particles 15.

The bonding-target P-particle(s) 16 may be arbitrarily selected from the P-particles 6 of each polymer model 5.

As shown in Fig. 4, in the second embodiment, similarly to the first embodiment, one P-particle 6 positioned at the almost center in the length of the straight chain arrangement of the P-particles 6 of each polymer model 5 is the bonding-target P-particle 16.

In the second embodiment, as shown in Fig. 19, one P-particle 18 positioned at one end of the straight chain arrangement of the P-particles 18 of each coupling agent model 17 is selected as the bonding-target C-particle 19.

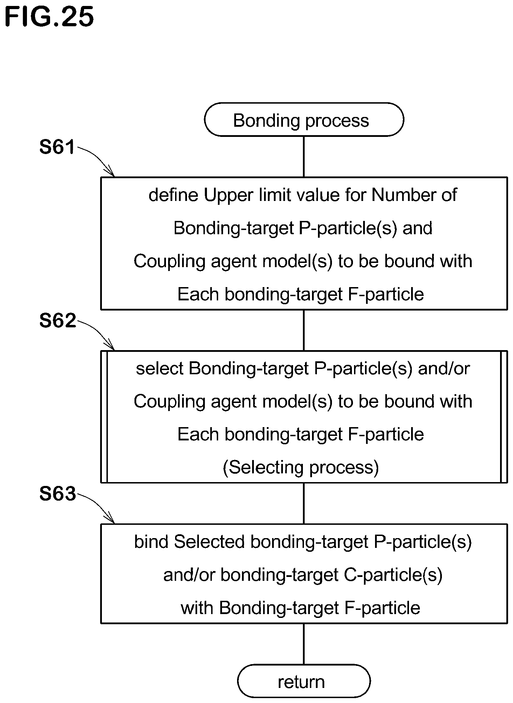

Fig. 25 is a flow chart of the bonding process S54 in the second embodiment.

* Process S61

In the bonding process S54, there is defined an upper limit value Ns for the sum (Sa + Sb) of the number Sa of the bonding-target P-particle(s) 16 and the number Sb of the bonding-target C-particle(s) 19 to be bound with each bonding-target F-particle 15. (Process S61)

The upper limit value Ns is determined depending on

the total number Na of the bonding-target P-particle 16 existing in the virtual space 9,
the total number Nb of the bonding-target F-particles 15 existing in the virtual space 9, and
the total number Nc of the bonding-target C-particle 19 existing in the virtual space 9.

According to the present invention, the upper limit value Ns is defined by the following expression (3).

$$Ns = [\ (Na+Nc)/Nb+1\ ]\ \ ---\ (3)$$

The total number Na is equal to the total number (in this example, 1000) of the polymer models 5 disposed in the virtual space 9.

The total number Nb is equal to the total number of the filler models 3 (=100) multiplied by the number of the bonding-target F-particles 15 per a filler model 3 (=8), namely 800.

In the second embodiment, the total number Nc is equal to the total number of the bonding-target C-particles 19 disposed in the virtual space 9, namely, 1000, since each coupling agent model 17 has a single bonding-target C-particle 19 as shown in Fig. 19.

Thus, the (Na+Nc)/Nb means the number of the particles (16, 19) per one bonding-target F-particle 15 when the bonding-target P-particles 16 and the bonding-target C-particles 19 are evenly bound with the bonding-target F-particles 15.

In the expression (3), the [ (Na+Nc)/Nb+1 ] means an integer obtained by rounding up the value (Na+Nc)/Nb.

For example, if Na=1000, Nb=800 and Nc=1000, then (Na+Nc)/Nb=2.5 and Ns=[ (Na+Nc)/Nb+1 ]=3.

* Selecting process S62

In the bonding process S54 in the second embodiment, the bonding-target P-particle(a) 16 and/or bonding-target C-particle(s) 19 to be bound with each bonding-target F-particle 15 are selected. (Selecting process S62)

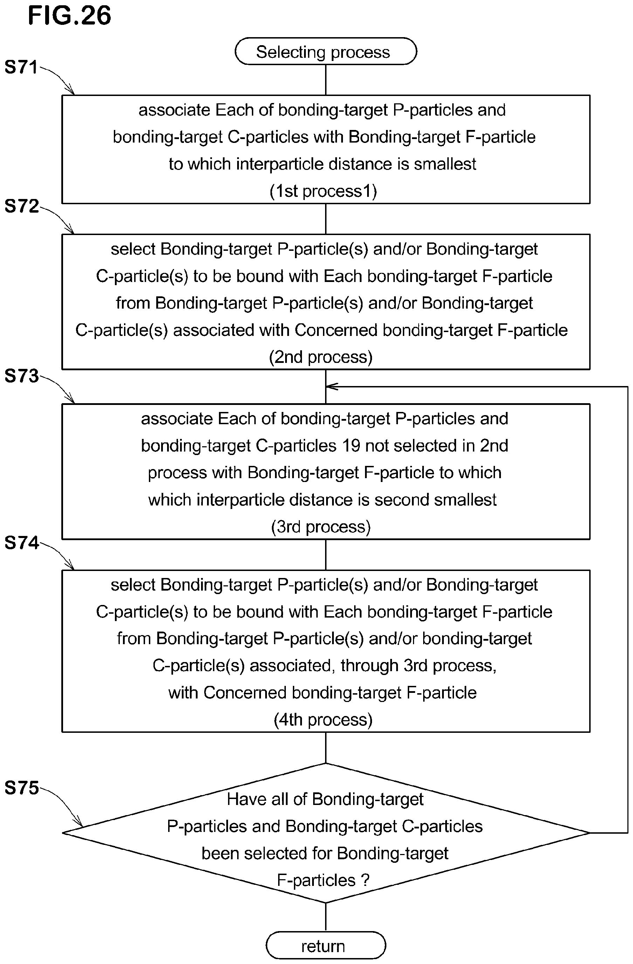

Fig. 26 is a flow chart of the selecting process 562.

* 1st process 571

In the selecting process S62, first, each of the bonding-target P-particles 16 and bonding-target C-particles 19 is associated with one bonding-target F-particle 15 to which interparticle distance Lp is smallest. (1st process S71)

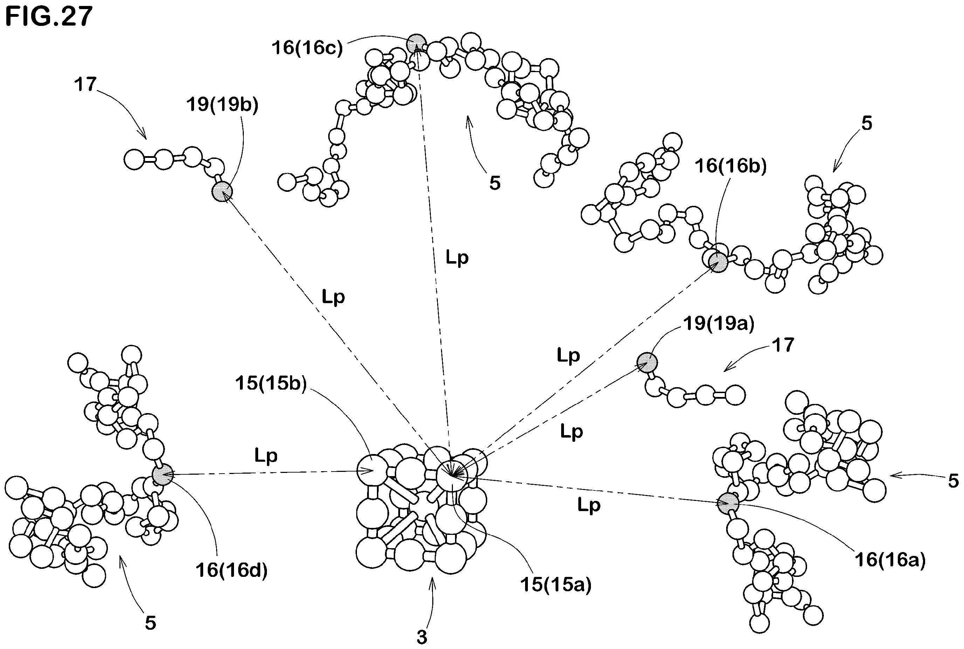

Fig. 27 is a diagram for explaining this process S71.

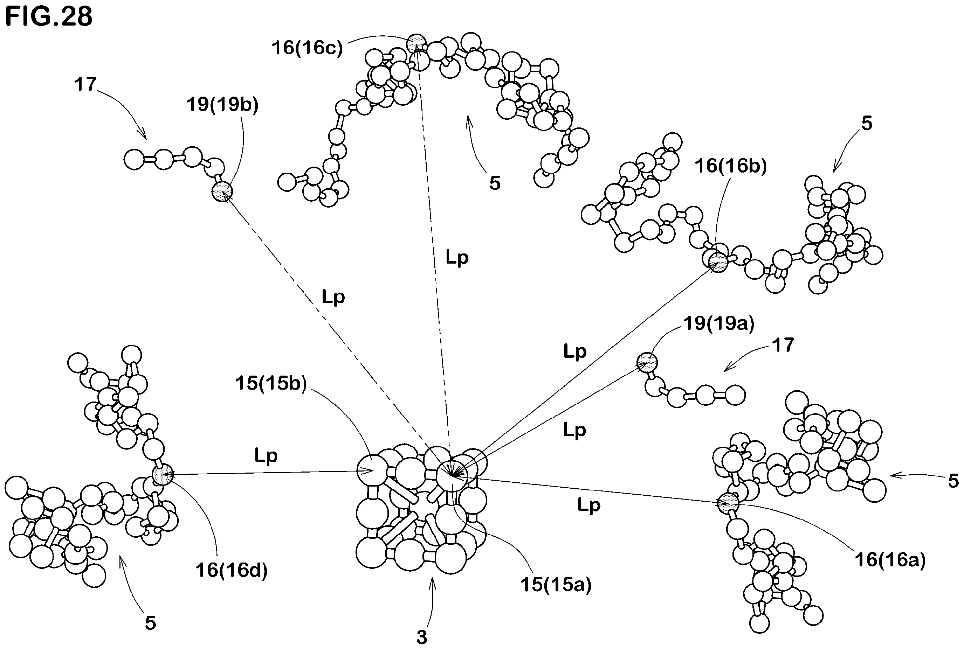

In Fig. 27 and the after-mentioned Fig. 28 to Fig. 31, the polymer models 5 bound with the coupling agent models 17 are omitted.

In the 1st process S71, for each bonding-target P-particle 16 in the virtual space 9 (shown in Fig. 22),

a bonding-target F-particle 15 to which interparticle distance Lp from the concerned bonding-target P-particle 16 is smallest is searched, and
the searched-out bonding-target F-particle 15 is associated with the concerned bonding-target P-particle 16.

In the 1st process S71, further, for each bonding-target C-particle 19 in the virtual space 9 (shown in Fig. 22),

a bonding-target F-particle 15 to which interparticle distance Lp from the concerned bonding-target C-particle 19 is smallest is searched, and
the searched-out bonding-target F-particle 15 is associated with the concerned bonding-target C-particle 19.

The interparticle distance Lp is the distance between the centers of the particles concerned.

Data about such associations, for example, identification numbers of the bonding-target P-particles 16, the bonding-target C-particles 19 and the bonding-target F-particles 15, and their coordinate values in relation to the virtual space 9, are stored in the computer 1.

In the example shown in Fig. 27, a first bonding-target F-particle 15a and a second bonding-target F-particle 15b are the bonding-target F-particles 15.

As to the first bonding-target F-particle 15a, three bonding-target P-particles 16 and two bonding-target C-particles 19 are associated therewith.

The three bonding-target P-particles 16 are a first bonding-target P-particle 16a, a second bonding-target P-particle 16b, and a third bonding-target P-particle 16c in the ascending order of the interparticle distance Lp from the bonding-target F-particle 15a.

The two bonding-target C-particles 19 are a first bonding-target C-particle 19a, and a second bonding-target C-particle 19b in the ascending order of the interparticle distance Lp from the bonding-target F-particle 15a.

As to the second bonding-target F-particle 15b, a single bonding-target P-particle 16 (referred to as the fourth bonding-target P-particle 16d) is associated therewith.

In Fig. 27, the associations of the bonding-target F-particles 15 with the bonding-target P-particles 16 and bonding-target C-particles 19 are respectively indicated by chain double-dashed lines.

* 2nd process S72

In the selecting process S62 in the second embodiment, next, the bonding-target P-particle(s) 16 and/or bonding-target C-particle(s) 19 to be bound with each bonding-target F-particle 15 are selected from the bonding-target P-particle(s) 16 and/or the bonding-target C-particle(s) 19 associated with the concerned bonding-target F-particle 15. (2nd process S72)

The selection is made in the ascending order of the interparticle distance Lp so that the sum (Sa+Sb) of the number Sa of the selected bonding-target P-particle(s) 16 and the number Sb of the selected bonding-target C-particle(s) 19 does not exceed the upper limit value Ns.

Fig. 28 is a diagram for explaining the process S72.

In this example, the upper limit value Ns is 3.

In the example shown in Fig. 27, the total number (=5) of the bonding-target P-particles 16 and the bonding-target C-particles 19 associated with the first bonding-target F-particle 15a is over the upper limit value Ns (=3).

Therefore, from the bonding-target P-particles 16 and bonding-target C-particles 19 associated with the first bonding-target F-particle 15a, regardless of whether P-particle 16 or C-particle 19, particles (16, 19) are selected in the ascending order of the interparticle distance Lp so that the number of the selected particles (16, 19) does not exceed the upper limit value Ns (in this example, 3).

In this example, the number of the selected particles (16, 19) is equal to Ns, and the first bonding-target P-particle 16a, the second bonding-target P-particle 16b, and the first bonding-target C-particle 19a are selected.

The selected particles (16a, 16b and 19a) are defined as particles to be bound with the first bonding-target F-particle 15a.

In Fig. 28, indicated by a solid line instead of the above-mentioned chain double-dashed line (Fig. 27) is a selected association.

The not-selected particles (16c and 19b) are defined as particles not to be bound with the first bonding-target F-particle 15a.

As to the second bonding-target F-particle 15b, on the other hand, the number (in this example, =1) of the fourth bonding-target P-particle 16d associated with the second bonding-target F-particle 15b is under the upper limit value Ns (=3).

Therefore, as shown in Fig. 28, the fourth bonding-target P-particle 16d is defined as a particle to be bound with the second bonding-target F-particle 15b.

In the 2nd process S72, therefore, the bonding-target P-particle(s) 16 and/or bonding-target C-particle(s) 19 is/are selected so that the number of the bonding-target P-particle(s) 16 and/or bonding-target C-particle(s) 19 to be bound with each bonding-target F-particle 15 does not exceed the upper limit value Ns.

Data about such selections, for example, identification numbers of the bonding-target P-particles 16, the bonding-target C-particles 19 and the bonding-target F-particles 15 and their coordinate values in relation to the virtual space 9, are stored in the computer 1.

* Third process s73

In the selecting process S62 in the second embodiment, next, each of the bonding-target P-particles 16 and bonding-target C-particles 19 not selected in the 2nd process S72 is associated with another bonding-target F-particle 15.

(Third process 573)

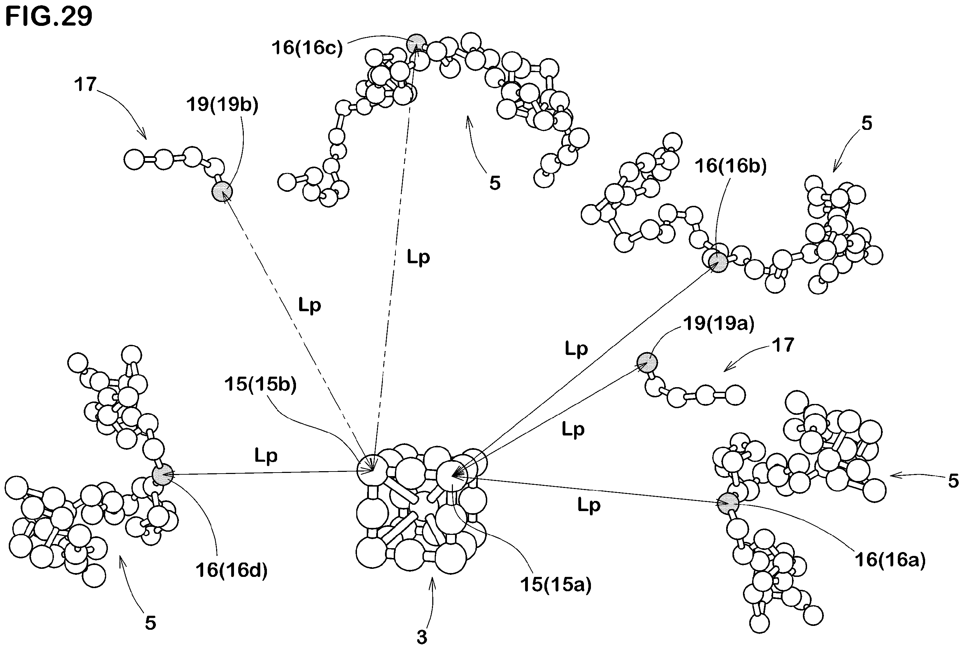

Fig. 29 is a diagram for explaining this process S73.
In the process S73, for each of the bonding-target P-particles 16 and bonding-target C-particles 19 not selected in the 2nd process S72,

a bonding-target F-particle 15 to which interparticle distance Lp from the concerned not-selected particle (16, 19) is second smallest next to the interparticle distance Lp of the bonding-target F-particle 15 associated with the concerned not-selected particle (16, 19) through the 1st process S71,
is searched, and
the searched-out bonding-target F-particle 15 is associated with the concerned not-selected particle (16, 19).

Data about such association are stored in the computer 1.
In the example shown in Fig. 28, the third bonding-target P-particle 16c and the second bonding-target C-particle 19b are the concerned not-selected particle (16, 19). And, the second bonding-target F-particle 15b is the searched-out bonding-target F-particle 15.
Therefore, as shown in Fig. 29, the third bonding-target P-particle 16c and the second bonding-target C-particle 19b are associated with the second bonding-target F-particle 15b.

* Fourth process S74

In the selecting process S62 in the second embodiment, next, the bonding-target P-particle(s) 16 and/or bonding-target C-particle(s) 19 to be bound with each bonding-target F-particle 15 is(are) selected from the bonding-target P-particle(s) 16 and/or bonding-target C-particle(s) 19 associated, through the third process S73, with the concerned bonding-target F-particle 15.

(Fourth process S74)

In the fourth process S74, similarly to the 2nd process S72, the selection is made in the ascending order of the interparticle distance Lp so that the sum (Sa+Sb) of the number Sa of the selected bonding-target P-particle(s) 16 and the number Sb of the selected bonding-target C-particle(s) 19 satisfies the upper limit value Ns, namely, does not render the total number of the resultant bound particles over the upper limit value Ns.

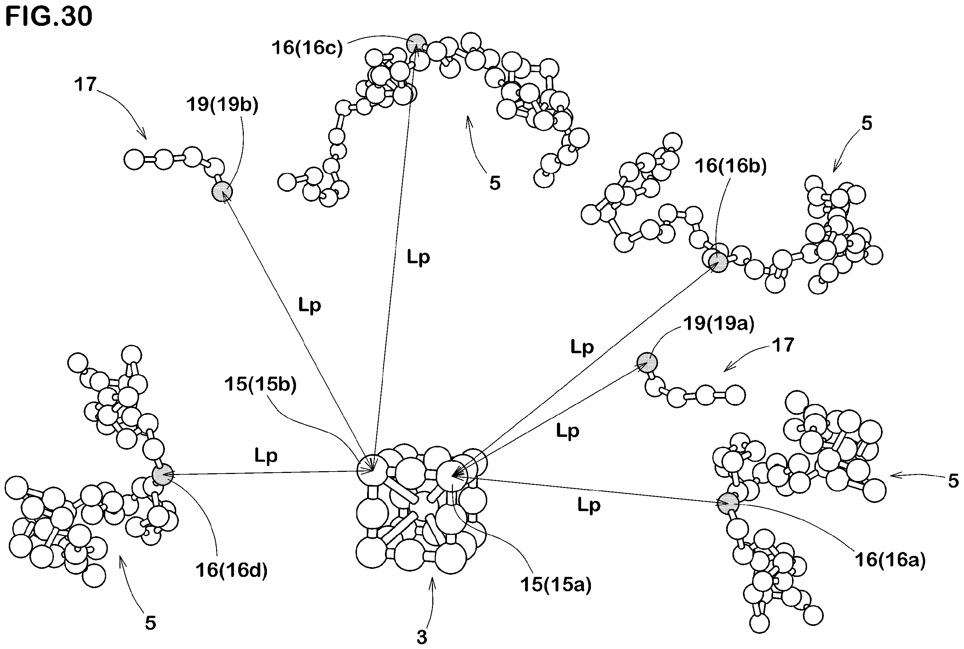

Fig. 30 is a diagram for explaining the fourth process S74. In this example, the third bonding-target P-particle 16c and the second bonding-target C-particle 19b are associated with the second bonding-target F-particle 15b with which the single bonding-target P-particle 16d has already been bound as shown in Fig. 29.
In the case of the second bonding-target F-particle 15b, therefore, the sum (Sa+Sb) satisfies the upper limit value Ns (=3 in this example).
Consequently, as shown in Fig. 30, the third bonding-target P-particle 16c and the second bonding-target C-particle 19b are each defined as a particle to be bound with the second bonding-target F-particle 15b.
If three or more bonding-target particles 16 and/or 19 are associated with the second bonding-target F-particle 15b, they are selected in the ascending order of the interparticle distance Lp so that the sum (Sa+Sb) satisfies the upper limit value Ns (=3 in this example).
Therefore, each of the P-particle(s) 16 and/or the C-particle(s) 19 to which interparticle distance Lp is the third smallest or more, is not defined as a particle to be bound with the second bonding-target F-particle 15b.
Thus, in the fourth process S74, the bonding-target P-particle(s) 16 and bonding-target C-particle(s) 19 to be bound with each bonding-target F-particle 15 are selected from the bonding-target P-particle(s) 16 and/or the bonding-target C-particle(s) 19 associated through the third process S73 so that the sum (Sa+Sb) does not render the total number of the resultant bound particles over the upper limit value Ns.
Data about the selections of the bonding-target P-particles 16 and the bonding-target C-particles 19 are stored in the computer 1.

*Process S75*

In the selecting process S62 in the second embodiment, next, it is checked whether or not all of the bonding-target P-particles 16 and the bonding-target C-particles 19 have been selected for the bonding-target F-particles 15. (Process S75)

If all of of them have been selected, the next process 563 is performed.

If not selected, with respect to the not selected particle(s) 16 and/or 19, the third process S73 and the fourth process S74 are repeated.

Thus, in the selecting process S62, all of the bonding-target P-particles 16 and the bonding-target C-particles 19 are selected for the bonding-target F-particles 15.

But, in this embodiment, there is a possibility of existence of a bonding-target F-particle 15 for which none of the bonding-target P-particles 16 and the bonding-target C-particles 19 is selected finally.

*Process 563*

Next, the bonding-target P-particle(s) 16 and/or bonding-target C-particle(s) 19 selected in the bonding process S46 are bound with the bonding-target F-particle 15.

(Process S63)

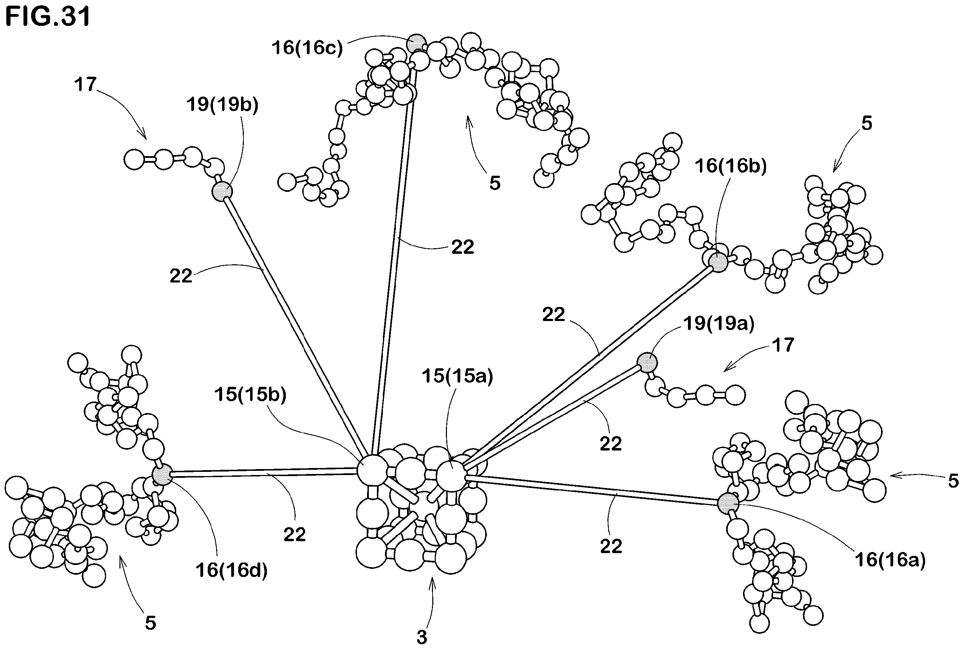

Fig. 31 is a diagram for explaining the process 563.

In the process S63, each selected bonding-target P-particle 16 is bound with the bonding-target F-particle 15 via a link 22 so that the filler models 3 and the polymer models 5 can simulate chemical bond between the filler and the polymer. Further, each selected bonding-target C-particle 19 is bound with the bonding-target F-particle 15 via a link 22 so that the filler models 3 and the coupling agent models 17 can simulate chemical bond between the filler and the coupling agent.

*Process 555*

In the simulation process S4, next, a molecular dynamics calculation is performed. (Process S55)

In the process S55, the Newton's equation of motion is applied to all of the filler models 3, the polymer models 5 and the coupling agent models 17, on the assumption that they accord with the classical dynamics for a specified period of time in the virtual space 9.

And the F-particles 4c and 4s (shown in Fig. 3), the P-particles 6a and 6b (shown in Fig. 4) and the C-particles 18 are traced to obtain their coordinates at each clock time.

Through the process S55, the filler models 3, the polymer models 5 and the coupling agent models 17 are distributed within the virtual space 9 as shown in Fig. 17.

As explained above, in the bonding process S46 in the simulation process S4 in the second embodiment, with respect to each bonding-target F-particle 15, the sum (Sa+Sb) of the number Sa of the bonding-target P-particle(s) 16 to be bound therewith and the number Sb of the bonding-target C-particle(s) 19 to be bound therewith is set to a value not more than the predetermined upper limit value Ns. Therefore, it is possible to prevent the occurrence of large variation in the number of the P-particle(s) 6 and C-particle(s) 18 bound with each F-particle 4. Thereby, in the simulation process S4 in the second embodiment, it is possible to prevent an excessive increase in the summation of the forces of the bonding-target P-particle(s) 16 and bonding-target C-particle(s) 19 exerted on each bonding-target F-particle 15 during the molecular dynamics calculation. Accordingly, in the simulation method according to the second embodiment, it is possible to avoid the occurrence of calculation failure to continue the molecular dynamics calculation stably.

In the second embodiment, as the upper limit value Ns is defined so as to satisfy the expression (3), based on the number of the particles (16, 19) per one bonding-target F-particle 15 when the bonding-target P-particles 16 and the bonding-target C-particles 19 are evenly bound with the bonding-target F-particles 15, the bonding-target P-particles 16 and the bonding-target C-particles 19 can be evenly bound with the bonding-target F-particles 15, and it is possible to prevent an excessive increase in the summation of the forces exerted on each bonding-target F-particle 15 during the molecular dynamics calculation.

In the second embodiment, since the bonding-target F-particles 15 are only the apex F-particles 7 disposed at the apexes 13a of the polyhedron 13 (shown in Fig. 3), it is possible to provide a space between the bonding-target P-particle(s) 16 or bonding-target C-particle(s) 19 bound with a bonding-target F-particle 15 and the bonding-target P-particle(s) 16 or bonding-target C-particle(s) 19 bound with an adjacent bonding-target F-particle 15. Thereby, it is possible to prevent the interaction between the bonding-target P-particle(s) 16 and/or bonding-target C-particle(s) 19 from becoming increased, and it is possible to stably perform the molecular dynamics calculation.

* Process S56

In the simulation process S4, next, it is judged with the computer 1 whether the filler models 3, the polymer models 5 and the coupling agent models 17 have been sufficiently distributed or not. (Process S56)

If sufficiently distributed ("Y" in the process S56), the next estimating process S5 (Fig. 2) is performed.

If not yet sufficiently distributed ("N" in the process S56), then a unit time step is incremented (process 549), and the process S55 is repeated.

Therefore, in the simulation process S4 in the second embodiment, a structurally-relaxed high-polymer material model 11 in which the filler models 3 and the polymer models 5 are effectually distributed, can be defined.

In order to effectually disperse the filler models 3, the polymer models 5 and the coupling agent models 17, the number of steps of the molecular dynamics calculation performed in the process S55, is preferably not less than 1000, more preferably not less than 5000 but preferably not more than 1000000 if the time interval of one step is 0.05[ $\tau$ ].

* Estimating process S5

In the simulation method according to the second embodiment, next, it is checked whether or not the dispersion state of the filler models 3 is goof or not as shown in Fig. 2.

(Estimating process S5)

In the estimating process S5, similarly to the estimating process S5 in the first embodiment, the radial distribution function is computed with respect to the center F-particle 4c of each filler model 3, and
it is checked with the computer 1 whether the results of the radial distribution functions are within the predetermined permissible limits or not.

If within the permissible limits, namely, the dispersion state is good, then according to the high-polymer material model 11, an actual high-polymer material is manufactured. (Process S6)

If outside the permissible limits, namely, the dispersion state is not good, then the conditions previously set to the filler models 3 and the polymer models 5 are changed, for example, taking the results of the radial distribution functions into account (process S7), and then the processes S1 to S5 are repeated.

Thereby, in the simulation method according to the second embodiment, the structure of the high-polymer material model 11 (shown in Fig. 17) can be effectively relaxed to simulate a structure of an actual high-polymer material.

In the simulation method according to the second embodiment, the structures of the filler models 3 and the polymer models 5 and the potentials can be arbitrarily defined, therefore, it is possible to estimate the properties and performances of a nonexistent unknown high-polymer material.

while description has been made of particularly preferable embodiments of the present invention, the illustrated embodiments should not be construed as to limit the scope of the present invention

Comparison Tests

[ Embodiment A ]

According to the procedures shown in Fig. 2, Fig. 9, Fig. 10 and Fig. 11, bonding-target P-particles were bound with bonding-target F-particles to satisfy the predetermined upper limit value Su, and the molecular dynamics calculation was performed. (Embodiment 1)

For comparison, bonding-target P-particles were bound with bonding-target F-particles randomly without the limitation to the upper limit value Su, and the molecular dynamics calculation was performed. (Comparative example 1)

Each of a simulation method as Embodiment 1 and a simulation method as Comparative example 1 was executed thirty times in order to check whether or not calculation failure occured during the molecular dynamics calculation.

Each parameter was as is written in the description.

Common specifications are as follows:

filler models:

total number: 100
number of bonding-target F-particles of each filler
model: 8

polymer models:

    total number: 2000
    number of bonding-target P-particle of each polymer
    model: 1

total number Na of bonding-target P-particle: 2000
total number Nb of bonding-target F-particles: 800
Na/Nb: 2.5
upper limit value Su: 2

In the simulation method as Comparative example 1, calculation failure was occurred during 15-time executions per 30-time executions.

In the simulation method as Embodiment 1, calculation failure was not occurred during 30-time executions and it was confirmed that the molecular dynamics calculation can be stably performed.

[ Embodiment B ]

According to the procedures shown in Fig. 18, Fig. 23, Fig. 25 and Fig. 26, the bonding-target P-particles and bonding-target C-particles were bound with the bonding-target F-particle to satisfy the predetermined upper limit value Ns, and the molecular dynamics calculation was performed. (Embodiments 2 to 4)

In the Embodiments 2-4, the numbers of the C-particles and the coefficients $a_{ij}$ for the potentials U11 to U14 were as follows.

Embodiment 2:

    number of C-particles: 5
    coefficients $a_{ij}$ for potentials U11 to U14: 50

Embodiment 3:

    number of C-particles: 1
    coefficients $a_{ij}$ for potentials U11 to U14: 50

Embodiment 4:

    number of C-particles: 5
    coefficient $a_{ij}$ for potential U11: 10
    coefficient $a_{ij}$ for potential U12: 10
    coefficient $a_{ij}$ for potential U13: 100
    coefficient $a_{ij}$ for potential U14: 100

Thus, in Embodiment 4, the affinity of the C-particle for the F-particle was defined as being higher than the affinity of C-particle for the P-particle.

For comparison, bonding-target P-particles and bonding-target C-particles were bound with bonding-target F-particles randomly without the limitation to the upper limit value Ns and the molecular dynamics calculation was performed. (Comparative example 2)

Each of simulation methods as Embodiments 2 to 4 and a simulation method as Comparative example 2 was executed thirty times in order to check whether or not calculation failure occured during the molecular dynamics calculation.

Except for the above specifications for the C-particle, each parameter was as is written in the description.

Common specifications are as follows:

total number Na of bonding-target P-particles: 1000
total number Nb of bonding-target F-particles: 800
total number Nc of bonding-target C-particles: 2500
(Na+Nc)/Nb+1: 5.375
upper limit value Ns: 6

In the embodiments 2 to 4, calculation failure was not occurred and the molecular dynamics calculation was made stably. In the comparative example 2, the molecular dynamics calculation could not be completed due to calculation failure.

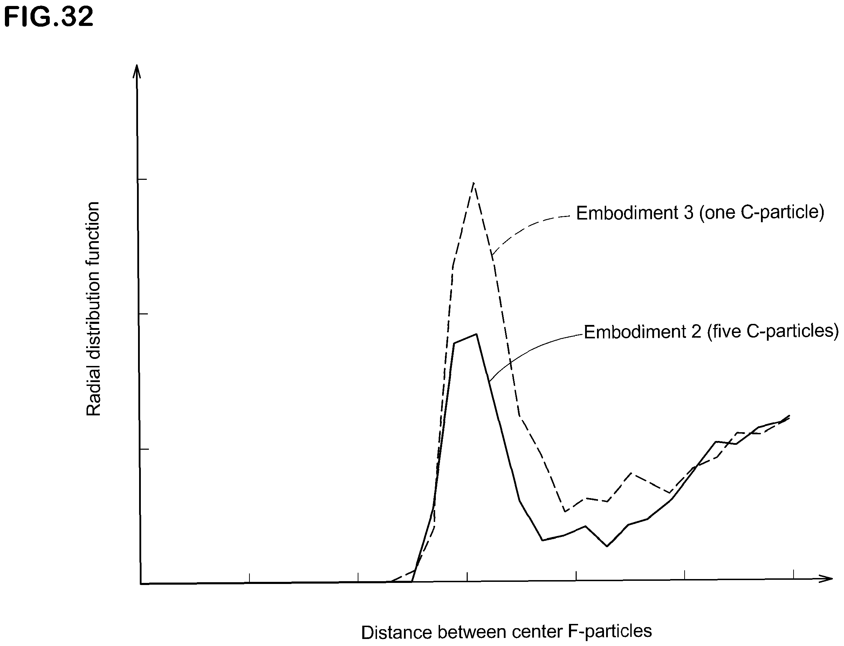

Radial distribution functions of Embodiments 2 and 3 are shown in Fig. 32.

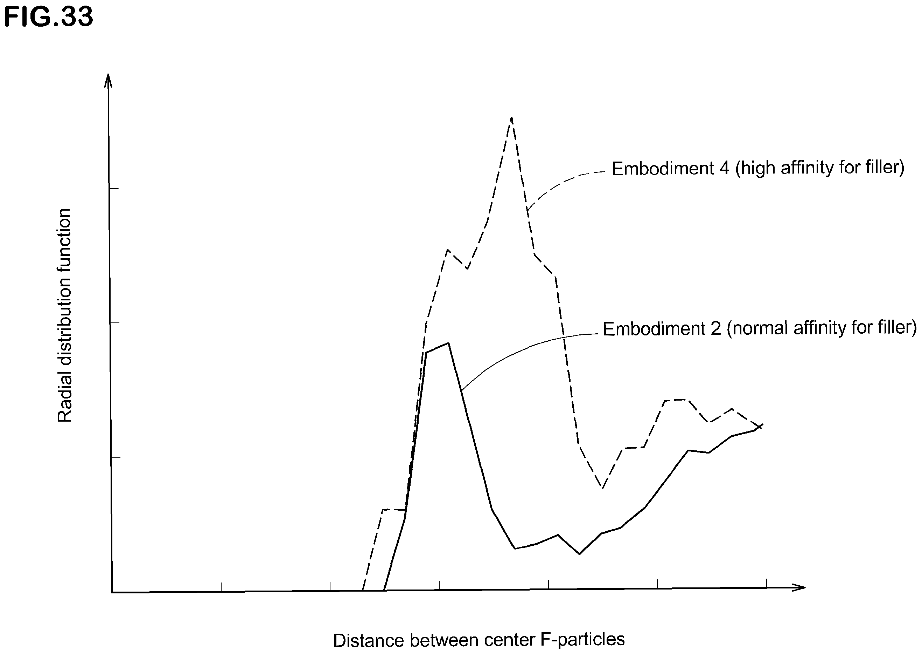

Radial distribution functions of Embodiments 2 and 4 are shown in Fig. 33.

In Fig. 32, the radial distribution of Embodiment 2 is smaller than the radial distribution of Embodiment 3, which means that the dispersion of the filler models of Embodiment 2 was better than that of the filler models of Embodiment 3.

It has been known in the art that, in general, if the chain length of a coupling agent is longer, the degree of dispersion of filler becomes higher.

Therefore, the simulation method according to the present invention can accurately simulate and estimate the influence of the chain length of the coupling agent on the dispersion of the filler.

In Fig. 33, the radial distribution of Embodiment 2 is smaller than the radial distribution function of Embodiment 4, which means that the dispersion of the filler models of Embodiment 2 was better than that of the filler models of Embodiment 4.

It has been known in the art that, in general, if a coupling agent whose affinity for the filler is the same as that for the polymer, the degree of dispersion of filler becomes high. Therefore, the simulation method according to the present invention can accurately simulate and estimate the influence of the interaction of the coupling agent on the dispersion of the filler.

Reference Signs List

1 computer
3 filler model
4 F-particle
5 polymer model
6 P-particle
9 virtual space
15 bonding-target F-particle
16 bonding-target P-particle

**Claims**

1. A simulation method for estimating dispersion of filler contained in a high-polymer material by the use of a computer, comprising:

    a process for defining filler models of the filler, wherein each filler model is a numerical model made up of a plurality of F-particles,
    a process for defining polymer models of the high-polymer material, wherein each polymer models is a numerical model made up of a plurality of P-particles, and
    a simulation process for executing a molecular dynamics calculation by the use of the filler models and the polymer models disposed in a predetermined virtual space,
    the simulation process including a bonding process for binding bonding-target P-particles selected from the P-particles with bonding-target F-particles selected from the F-particles, wherein

    the number of the bonding-target P-particle(s) to be bound with each bonding-target F-particle satisfies a predetermined upper limit value Su, wherein
    the upper limit value Su, the total number Na of the bonding-target P-particles existing in the virtual space, and the total number Nb of the bonding-target F-particles existing in the virtual space satisfy the following expression:

$$Na/Nb-1 =< Su =< Na/Nb+1.$$

2. The simulation method according to claim 1, wherein
the bonding process includes:

    a process for defining the upper limit value Su,
a selecting process for selecting the bonding-target P-particle(s) to be bound with each bonding-target F-particle, and
a process for binding the selected bonding-target P-particle(s) with the concerned bonding-target F-particle.

3. The simulation method according to claim 2, wherein
the selecting process includes:

    a 1st process for associating each bonding-target P-particle with a bonding-target F-particle to which interparticle distance is smallest,
a 2nd process for selecting the bonding-target P-particle(s) to be bound with each bonding-target F-particle from a group of the bonding-target P-particle(s) associated with the concerned bonding-target F-particle in the ascending order of the interparticle distance to satisfy the upper limit value Su,
a third process for associating each bonding-target P-particle not selected in the 2nd process with a bonding-target F-particle to which interparticle distance is second smallest next to said bonding-target F-particle to which interparticle distance is smallest, and
a fourth process for selecting

        the bonding-target P-particle(s) to be bound with each bonding-target F-particle
from a group of the bonding-target P-particle(s) associated in the third process,
in the ascending order of the interparticle distance to satisfy the upper limit value Su, and

    the third process and the fourth process are repeated until all of the bonding-target P-particles are selected for the bonding-target F-particles.

4. A simulation method for estimating dispersion of filler contained in a high-polymer material together with a coupling agent for coupling the filler with polymer by the use of a computer, comprising:

    a process for defining filler models of the filler, wherein each filler model is a numerical model made up of a plurality of F-particles,
a process for defining polymer models of the high-polymer material, wherein each polymer models is a numerical model made up of a plurality of P-particles, and
a process for defining coupling agent models of the coupling agent, wherein each coupling agent model is a numerical model made up of at least one C-particle,
a simulation process for executing a molecular dynamics calculation by the use of the filler models, the coupling agent models and the polymer models disposed in a predetermined virtual space,
the simulation process including a bonding process in which bonding-target P-particles selected from the P-particles and bonding-target C-particles selected from the C-particles are bound with bonding-target F-particles selected from the F-particles,
wherein
the number of the bonding-target P-particle(s) and/or the bonding-target C-particle(s) to be bound with each bonding-target F-particle satisfies a predetermined upper limit value Ns,
wherein the upper limit value Ns, the total number Na of the bonding-target P-particles existing in the virtual space, the total number Nb of the bonding-target F-particles existing in the virtual space, and the total number Nc of the bonding-target C-particles existing in the virtual space satisfy the following expression:

$$Ns = [(Na+Nc)/Nb+1].$$

5. The simulation method according to claim 4, wherein
the bonding process includes:

EP 2 933 744 B1

a process for defining the upper limit value Ns,
a selecting process for selecting the bonding-target P-particle(s) and/or the bonding-target C-particle(s) to be bound with each bonding-target F-particle, and
a process for binding the selected bonding-target P-particle(s) and/or bonding-target C-particle(s) with the concerned bonding-target F-particle.

6. The simulation method according to claim 5, wherein
the selecting process includes:

a 1st process for associating each bonding-target P-particle with a bonding-target F-particle to which interparticle distance is smallest, and associating each bonding-target C-particle with a bonding-target F-particle to which interparticle distance is smallest,
a 2nd process for selecting

the bonding-target P-particle(s) and/or the bonding-target C-particle(s) to be bound with each bonding-target F-particle from the associated bonding-target P-particle(s) and/or bonding-target C-particle(s)
in the ascending order of the interparticle distance to satisfy the upper limit value Ns,

a third process for

associating each bonding-target P-particle not selected in the 2nd process with a bonding-target F-particle to which interparticle distance is second smallest next to said bonding-target F-particle to which interparticle distance is smallest, and
associating each bonding-target C-particle not selected in the 2nd process with a bonding-target F-particle to which interparticle distance is second smallest next to said bonding-target F-particle to which interparticle distance is smallest, and

a fourth process for selecting

the bonding-target P-particle(s) and/or the bonding-target C-particle(s) to be bound with each bonding-target F-particle from the bonding-target P-particle(s) and/or the bonding-target C-particle(s) associated in the third process
in the ascending order of the interparticle distance to satisfy the upper limit value Ns, and

the third process and the fourth process are repeated until all of the bonding-target P-particles and the bonding-target C-particles are selected for the bonding-target F-particles.

7. The simulation method according to any one of claims 1-6, wherein
the F-particles of each filler model include those disposed at apexes of a polyhedron, and the bonding-target F-particles are the F-particles disposed at the apexes.


**Patentansprüche**

1. Simulationsverfahren zum Schätzen der Dispersion von Füllstoff, der in einem hochpolymeren Material enthalten ist, unter Verwendung eines Computers, umfassend:

einen Prozess zum Definieren von Füllstoffmodellen des Füllstoffes, wobei jedes Füllstoffmodell ein numerisches Modell ist, das aus einer Mehrzahl an F-Partikeln aufgebaut ist,
einen Prozess zum Definieren von Polymermodellen des hochpolymeren Materials, wobei jedes Polymermodell ein numerisches Modell ist, das aus einer Mehrzahl an P-Partikeln aufgebaut ist, und
einen Simulationsprozess zum Ausführen einer Molekulardynamikrechnung unter Verwendung der in einem vorgegebenen virtuellen Raum angeordneten Füllstoffmodelle und Polymermodelle,
wobei der Simulationsprozess einen Bindungsprozess zum Binden aus den P-Partikeln ausgewählter Bindungs-Ziel-P-Partikel an aus den F-Partikeln ausgewählte Bindungs-Ziel-F-Partikel umfasst, wobei die Anzahl der/des an jedes Bindungs-Ziel-F-Partikel zu bindenden Bindungs-Ziel-P-Partikel(s) einen vorgegebenen oberen Grenzwert Su einhält, wobei
der obere Grenzwert Su, die Gesamtanzahl Na der in dem virtuellen Raum vorhandenen Bindungs-Ziel-P-

25

Partikel und die Gesamtanzahl Nb der in dem virtuellen Raum vorhandenen Bindungs-Ziel-F-Partikel dem folgenden Ausdruck genügen:

$$Na/Nb-1 =< Su =< Na/Nb+1.$$

2. Simulationsverfahren nach Anspruch 1, wobei der Bindungsprozess umfasst:

einen Prozess zum Definieren des oberen Grenzwerts Su,
einen Auswahlprozess zum Auswählen des/der an jeden Bindungs-Ziel-F-Partikel zu bindenden Bindungs-Ziel-P-Partikel(s), und
einen Prozess zum Binden des/der ausgewählten Bindungs-Ziel-P-Partikel(s) an das jeweilige Bindungs-Ziel-F-Partikel.

3. Simulationsverfahren nach Anspruch 2, wobei
der Auswahlprozess enthält:

einen 1. Prozess zum Verknüpfen jedes Bindungs-Ziel-P-Partikels mit einem Bindungs-Ziel-F-Partikel, zu dem der Zwischenpartikelabstand am kleinsten ist,
einen 2. Prozess zum Auswählen des/der an jedes Bindungs-Ziel-F-Partikel zu bindenden Bindungs-Ziel-P-Partikel(s) aus einer Gruppe des/der mit dem jeweiligen Bindungs-Ziel-F-Partikel verknüpften Bindungs-Ziel-P-Partikel(s) in aufsteigender Reihenfolge des Zwischenpartikelabstands, um den oberen Grenzwert Su einzuhalten,
einen dritten Prozess zum Verknüpfen jedes nicht im 2. Prozess ausgewählten Bindungs-Ziel-P-Partikels mit einem Bindungs-Ziel-F-Partikel, zu dem der Zwischenpartikelabstand am zweitkleinsten ist, nach demjenigen Bindungs-Ziel-F-Partikel, zu dem der Zwischenpartikelabstand am kleinsten ist, und
einen vierten Prozess zum Auswählen des/der an jedes Bindungs-Ziel-F-Partikel zu bindenden Bindungs-Ziel-P-Partikel(s) aus einer Gruppe des/der im dritten Prozess verknüpften Bindungs-Ziel-P-Partikel(s),

in aufsteigender Reihenfolge des Zwischenpartikelabstands,
um den oberen Grenzwert Su einzuhalten, und

der dritte Prozess und der vierte Prozess wiederholt werden, bis alle Bindungs-Ziel-P-Partikel für die Bindungs-Ziel-F-Partikel ausgewählt sind.

4. Simulationsverfahren zum Schätzen der Dispersion von Füllstoff, der in einem hochpolymeren Material enthalten ist, zusammen mit einem Kopplungsmittel zum Koppeln des Füllstoffes mit Polymer, unter Verwendung eines Computers, umfassend:

einen Prozess zum Definieren von Füllstoffmodellen des Füllstoffes, wobei jedes Füllstoffmodell ein numerisches Modell ist, das aus einer Mehrzahl an F-Partikeln aufgebaut ist,
einen Prozess zum Definieren von Polymermodellen des hochpolymeren Materials, wobei jedes Polymermodell ein numerisches Modell ist, das aus einer Mehrzahl an P-Partikeln aufgebaut ist, und
einen Prozess zum Definieren von Kopplungsmittelmodellen des Kopplungsmittels, wobei jedes Kopplungsmittelmodell ein numerisches Modell ist, das aus mindestens einem C-Partikel aufgebaut ist,
einen Simulationsprozess zum Ausführen einer Molekulardynamikrechnung unter Verwendung der in einem vorgegebenen virtuellen Raum angeordneten Füllstoffmodelle, Kopplungsmittelmodelle und Polymermodelle, wobei der Simulationsprozess einen Bindungsprozess umfasst, in welchem aus den P-Partikeln ausgewählte Bindungs-Ziel-P-Partikel und aus den C-Partikeln ausgewählte Bindungs-Ziel-C-Partikel an aus den F-Partikeln ausgewählte Bindungs-Ziel-F-Partikel gebunden werden, wobei
die Anzahl der/des an jedes Bindungs-Ziel-F-Partikel zu bindenden Bindungs-Ziel-P-Partikel(s) und/oder Bindungs-Ziel-C-Partikel(s) einen vorgegebenen oberen Grenzwert Ns einhält, wobei
der obere Grenzwert Ns, die Gesamtanzahl Na der in dem virtuellen Raum vorhandenen Bindungs-Ziel-P-Partikel, die Gesamtanzahl Nb der in dem virtuellen Raum vorhandenen Bindungs-Ziel-F-Partikel und die Gesamtanzahl Nc der in dem virtuellen Raum vorhandenen Bindungs-Ziel-C-Partikel dem folgenden Ausdruck genügen:

$$Ns = [(Na+Nc)/Nb+1].$$

**5.** Simulationsverfahren nach Anspruch 4, wobei der Bindungsprozess umfasst:

einen Prozess zum Definieren des oberen Grenzwerts Ns,
einen Auswahlprozess zum Auswählen des/der an jedes Bindungs-Ziel-F-Partikel zu bindenden Bindungs-Ziel-P-Partikel(s) und/oder Bindungs-Ziel-C-Partikel(s), und

einen Prozess zum Binden des/der ausgewählten Bindungs-Ziel-P-Partikel(s) und/oder Bindungs-Ziel-C-Partikel(s) an das jeweilige Bindungs-Ziel-F-Partikel.

**6.** Simulationsverfahren nach Anspruch 5, wobei der Auswahlprozess umfasst:

einen 1. Prozess zum Verknüpfen jedes Bindungs-Ziel-P-Partikels mit einem Bindungs-Ziel-F-Partikel, zu dem der Zwischenpartikelabstand am kleinsten ist, und zum Verknüpfen jedes Bindungs-Ziel-C-Partikels mit einem Bindungs-Ziel-F-Partikel, zu dem der Zwischenpartikelabstand am kleinsten ist,
einen 2. Prozess zum Auswählen

des/der an jedes Bindungs-Ziel-F-Partikel zu bindenden Bindungs-Ziel-P-Partikel(s) und/oder Bindungs-Ziel-C-Partikel(s) aus den verknüpften Bindungs-Ziel-P-Partikel(n) und/oder Bindungs-Ziel-C-Partikel(n) in aufsteigender Reihenfolge des Zwischenpartikelabstands,
um den oberen Grenzwert Ns einzuhalten,

einen dritten Prozess zum

Verknüpfen jedes nicht im 2. Prozess ausgewählten Bindungs-Ziel-P-Partikels mit einem Bindungs-Ziel-F-Partikel, zu dem der Zwischenpartikelabstand am zweitkleinsten ist, nach demjenigen Bindungs-Ziel-F-Partikel, zu dem der Zwischenpartikelabstand am kleinsten ist, und
Verknüpfen jedes nicht im 2. Prozess ausgewählten Bindungs-Ziel-C-Partikels mit einem Bindungs-Ziel-F-Partikel, zu dem der Zwischenpartikelabstand am zweitkleinsten ist, nach demjenigen Bindungs-Ziel-F-Partikel, zu dem der Zwischenpartikelabstand am kleinsten ist, und

einen vierten Prozess zum Auswählen des/der an jedes Bindungs-Ziel-F-Partikel zu bindenden Bindungs-Ziel-P-Partikel(s) und/oder Bindungs-Ziel-C-Partikel(s) aus dem/den im dritten Prozess verknüpften Bindungs-Ziel-P-Partikel(n) und/oder Bindungs-Ziel-C-Partikel(n) in aufsteigender Reihenfolge des Zwischenpartikelabstands, um den oberen Grenzwert Ns einzuhalten, und
der dritte Prozess und der vierte Prozess wiederholt werden, bis alle Bindungs-Ziel-P-Partikel und Bindungs-Ziel-C-Partikel für die Bindungs-Ziel-F-Partikel ausgewählt sind.

**7.** Simulationsverfahren nach einem der Ansprüche 1 bis 6, wobei die F-Partikel jedes Füllstoffmodells diejenigen umfassen, die an Spitzen eines Polyeders angeordnet sind, und die Bindungs-Ziel-F-Partikel die an den Spitzen angeordneten F-Partikel sind.

**Revendications**

**1.** Procédé de simulation pour estimer la dispersion d'une charge contenue dans un matériau à haut polymère par l'utilisation d'un ordinateur, comprenant :

un processus pour définir des modèles de charges de la charge, dans lequel chaque modèle de charge est un modèle numérique fait d'une pluralité de particules F,
un processus pour définir des modèles de polymères du matériau à haut polymère, dans lequel chaque modèle de polymère est un modèle numérique fait d'une pluralité de particules P, et
un processus de simulation pour exécuter un calcul de dynamique moléculaire par l'utilisation des modèles de charges et des modèles de polymères agencés dans un espace virtuel prédéterminé,

le processus de simulation incluant un processus de liaison pour lier des particules P cibles de liaison choisies parmi les particules P avec des particules F cibles de liaison choisies parmi les particules F, dans lequel le nombre de particules P cibles de liaison devant être liées avec chaque particule F cible de liaison satisfait à une valeur limite supérieure prédéterminée Su, dans lequel la valeur limite supérieure Su, le nombre total Na de particules P cibles de liaison existant dans l'espace virtuel, et le nombre total Nb de particules F cibles de liaison existant dans l'espace virtuel satisfait à l'expression :

$$Na/Nb - 1 \leq Su \leq Na/Nb + 1.$$

2. Procédé de simulation selon la revendication 1, dans lequel le processus de liaison comporte :

un processus pour définir la valeur limite supérieure Su,
un processus de sélection pour choisir la(les) particule(s) P cible(s) de liaison devant être liée(s) à chaque particule F cible de liaison, et
un processus pour lier la(les) particule(s) P cible(s) de liaison avec la particule F cible de liaison concernée.

3. Procédé de simulation selon la revendication 2, dans lequel le processus de sélection comporte :

un premier processus permettant d'associer chaque particule P cible de liaison avec une particule F cible de liaison par rapport à laquelle la distance interparticulaire est la plus petite,
un deuxième processus de sélection de la (des) particule(s) P cible(s) de liaison devant être liée(s) avec chaque particule F cible de liaison parmi un groupe de particule(s) P cible(s) de liaison associée(s) avec la particule F cible de liaison concernée dans l'ordre croissant de la distance interparticulaire afin de satisfaire la valeur limite supérieure Su,
un troisième processus d'association de chaque particule P cible de liaison non sélectionnée dans le deuxième processus avec une particule F cible de liaison par rapport à laquelle la distance interparticulaire est la deuxième plus petite par rapport à ladite particule F cible de liaison suivante par rapport à laquelle la distance interparticulaire est la plus petite, et
un quatrième processus de sélection de la (des) particule(s) P cible(s) de liaison devant être liée(s) avec chaque particule F cible de liaison parmi un groupe de particule(s) P cible(s) de liaison associée(s) dans le troisième processus, dans l'ordre croissant de la distance interparticulaire pour satisfaire la valeur limite supérieure Su, et le troisième processus et le quatrième processus sont répétés jusqu'à ce que toutes les particules P cibles de liaison soient sélectionnées pour les particules F cibles de liaison.

4. Procédé de simulation pour estimer la dispersion d'une charge contenue dans un matériau à haut polymère conjointement avec un agent de couplage permettant le couplage de la charge avec le polymère par l'utilisation d'un ordinateur, comprenant :

un processus pour définir des modèles de charges de la charge, dans lequel chaque modèle de charge est un modèle numérique fait d'une pluralité de particules F,
un processus pour définir des modèles de polymères du matériau à haut polymère, dans lequel chaque modèle de polymère est un modèle numérique fait d'une pluralité de particules P, et
un processus pour définir des modèles d'agents de couplage de l'agent de couplage, dans lequel chaque modèle d'agent de couplage est un modèle numérique fait d'au moins une particule C,
un processus de simulation pour exécuter un calcul de dynamique moléculaire par l'utilisation des modèles de charges, des modèles d'agents de couplage et des modèles de polymères agencés dans un espace virtuel prédéterminé,
le processus de simulation comportant un processus de liaison dans lequel des particules P cibles de liaison choisies parmi les particules P et des particules C cibles de liaison choisies parmi les particules C sont liées avec des particules F cibles de liaison choisies parmi les particules F,
dans lequel
le nombre de particules P cibles de liaison, et/ou de particules C cibles de liaison, devant être liées avec chaque particule F cible de liaison, satisfait une valeur limite supérieure Ns prédéterminée,
dans lequel
la valeur limite supérieure Ns, le nombre total Na de particules P cibles de liaison existant dans l'espace virtuel, le nombre total Nb de particules F cibles de liaison existant dans l'espace virtuel, et le nombre total Nc de particules C cibles de liaison existant dans l'espace virtuel satisfont l'expression suivante :

$$Ns = [(Na + Nc)/Nb + 1].$$

5. Procédé de simulation selon la revendication 4, dans lequel le processus de liaison comporte :

un processus pour définir la valeur limite supérieure Ns, un processus de sélection pour choisir la (les) particule(s) P cible(s) de liaison et/ou la (les) particule(s) C cible(s) de liaison devant être liée(s) avec chaque particule F cible de liaison, et
un processus de liaison pour lier la (les) particule(s) P cible(s) de liaison et/ou la (les) particule(s) C cible(s) de liaison avec la particule F cible de liaison concernée.

6. Procédé de simulation selon la revendication 5, dans lequel le processus de sélection comporte :

un premier processus pour associer chaque particule P cible de liaison avec une particule F cible de liaison par rapport à laquelle la distance interparticulaire est la plus faible, et associer chaque particule C cible de liaison avec une particule F cible de liaison par rapport à laquelle la distance interparticulaire est la plus faible,
un deuxième processus de sélection de la (des) particule(s) P cible(s) de liaison et/ou de la (des particule(s) C cible(s) de liaison devant être liée(s) avec chaque particule F cible de liaison parmi les particule(s) P cible(s) de liaison associée(s) et/ou particule(s) C cible(s) de liaison dans l'ordre croissant de la distance interparticulaire pour satisfaire la valeur limite supérieure Ns,
un troisième processus pour associer chaque particule P cible de liaison non sélectionnée dans le deuxième processus avec une particule F cible de liaison par rapport à laquelle la distance interparticulaire est la deuxième la plus petite jusqu'à ladite particule F cible de liaison par rapport à laquelle la distance interparticulaire est la plus petite, et
associer chaque particule C cible de liaison non sélectionnée dans le deuxième processus avec une particule F cible de liaison par rapport à laquelle la distance interparticulaire est la deuxième plus petite après ladite particule F cible de liaison pour laquelle la interparticulaire est la plus faible, et
un quatrième processus de sélection de la (des) particule(s) P cible(s) de liaison et/ou de la (des) particule(s) C cible(s) de liaison devant être reliée(s) avec chaque particule F cible de liaison parmi les particules P cibles de liaison et/ou les particules C cibles de liaison associées dans le troisième processus dans l'ordre croissant de la distance interparticulaire pour satisfaire la valeur limite supérieure Ns, et
le troisième processus et le quatrième processus sont répétés jusqu'à ce que toutes les particules P cibles de liaison et les particules C cibles de liaison soient sélectionnées pour les particules F cibles de liaison.

7. Procédé de simulation selon l'une quelconque des revendications 1 à 6, dans lequel
les particules F de chaque modèle de charge comportent celles disposées aux apex d'un polyèdre, et les particules F cibles de liaison sont les particules F disposées aux apex.

# FIG.1

# FIG.2

# FIG.3

FIG.4

# FIG.5

S31

Condition setting process

define Potentials
between F-particles and P-particles

S32

dispose Filler models and
Polymer models in Virtual space

S33

define Compact cluster of Filler models

return

# FIG.6

EP 2 933 744 B1

EP 2 933 744 B1

**FIG.7**

**FIG.8**

EP 2 933 744 B1

# FIG.9

```
                    ┌─────────────────────────┐
                    │    Simulation process    │
                    └─────────────────────────┘
                                 │
   S41 ┌───────────────────────────────────────────┐
       │        restrain Motion of each F-particle   │
       │         (Filler restraining process)        │
       └───────────────────────────────────────────┘
                                 │
   S42 ┌───────────────────────────────────────────┐ ◄──────────────┐
       │        perform Molecular dynamics           │                │
       │      calculation WRT Polymer models only     │      S45       │
       └───────────────────────────────────────────┘   ┌──────────────────────────────┐
                                 │                       │  increment a unit time step   │
   S43              ◇◇◇◇◇◇◇◇◇◇◇◇◇◇◇◇            N        └──────────────────────────────┘
              ◇  Have Polymer models   ◇ ──────────────────────┘
              ◇ been sufficiently distributed ? ◇
                     ◇◇◇◇◇◇◇◇◇◇◇◇
                                 │ Y
   S44 ┌───────────────────────────────────────────┐
       │              release F-particles            │
       └───────────────────────────────────────────┘
                                 │
   S46 ┌───────────────────────────────────────────┐
       │         bind F-particles with P-particles   │
       │              (Bonding process)              │
       └───────────────────────────────────────────┘
                                 │
   S47 ┌───────────────────────────────────────────┐ ◄──────────────┐
       │     perform Molecular dynamics calculation  │                │
       │      WRT Filler models and Polymer models    │      S49       │
       └───────────────────────────────────────────┘   ┌──────────────────────────────┐
                                 │                       │  increment a unit time step   │
   S48           ◇◇◇◇◇◇◇◇◇◇◇◇◇◇◇◇◇◇           N         └──────────────────────────────┘
              ◇   Have Filler models    ◇ ──────────────────────┘
              ◇  and Polymer models been  ◇
              ◇  sufficiently distributed ? ◇
                     ◇◇◇◇◇◇◇◇◇◇◇◇◇
                                 │ Y
                    ┌─────────────────────────┐
                    │         return           │
                    └─────────────────────────┘
```

# FIG.10

# FIG.11

Selecting process

S21

associate Each bonding-target P-particle with
Bonding-target F-particle to which interparticle
distance therefrom is smallest
(1st process)

S22

select, for Each bonding-target F-particle,
Bonding-target P-particle(s) to be bound therewith from
Group of Bonding-target P-particle(s) associated therewith
(2nd process)

S23

associate Each bonding-target P-particle not selected in
2nd process with Bonding-target F-particle to which
which interparticle distance therefrom is second smallest
(3rd process)

S24

select, for Each bonding-target F-particle, Bonding-target
P-particles to be bound therewith from Group of Bonding-target
P-particle(s) associated therewith in 3rd process
(4th process)

S25

Have all of Bonding-target
P-particles been selected for Bonding-target
F-particles ?

return

FIG.12

EP 2 933 744 B1

FIG.13

FIG.14

FIG.15

FIG.16

FIG.17

# FIG.18

start

**S1**

define Filler models

**S2**

define Polymer models

**S8**

define Coupling agent models

**S3**

define Simulation conditions

(Condition setting process)

**S4**

perform Molecular dynamics calculation

(Simulation process S4)

**S9**

change Conditions of

Filler models, Polymer models

and Coupling agent models

**S5**

Is dispersion state
of Filler models good ?
(Estimating process)

N

**S6**

Y

manufacture High-polymer material

end

## FIG.19

# FIG.20

Condition setting process

S31

define Potentials between
F-particles and P-particles

S34

define Potentials between Coupling
agent models and F-particles and
define Potentials between Coupling
agent models and P-particles

S32

dispose Filler models,
Polymer models and Coupling
agent models in Virtual space

S33

define Compact cluster of Filler models

return

FIG.21

EP 2 933 744 B1

FIG.22

EP 2 933 744 B1

# FIG.23

Simulation process

S41 —

restrain motion of each F-particle
(Filler restraining process)

S51 —

perform Molecular dynamics calculation WRT
Polymer models and Coupling agent models

S45

S52 —

Have Polymer models and
Coupling agent models been sufficiently
distributed ?  N

increment a unit time step

Y

S44 —

release F-particles

S53 —

bind P-particles with C-particles

S54 —

bind F-particles with P-particles and C-particles
(Bonding process)

S55 —

perform Molecular dynamics calculation
WRT Filler models, Polymer models
and Coupling agent models

S49

S56 —

Have Filler models,
Polymer models and Coupling agent models
been sufficiently distributed ?  N

increment a unit time step

Y

return

**FIG.24(a)**

**FIG.24(b)**

# FIG.25

Bonding process

S61
define Upper limit value for Number of
Bonding-target P-particle(s) and
Coupling agent model(s) to be bound with
Each bonding-target F-particle

S62
select Bonding-target P-particle(s) and/or
Coupling agent model(s) to be bound with
Each bonding-target F-particle
(Selecting process)

S63
bind Selected bonding-target P-particle(s)
and/or bonding-target C-particle(s)
with Bonding-target F-particle

return

## FIG.26

Selecting process

S71

associate Each of bonding-target P-particles and
bonding-target C-particles with Bonding-target F-particle
to which interparticle distance is smallest
(1st process1)

S72

select Bonding-target P-particle(s) and/or Bonding-target
C-particle(s) to be bound with Each bonding-target F-particle
from Bonding-target P-particle(s) and/or Bonding-target
C-particle(s) associated with Concerned bonding-target F-particle
(2nd process)

S73

associate Each of bonding-target P-particles and
bonding-target C-particles 19 not selected in 2nd
process with Bonding-target F-particle to which
which interparticle distance is second smallest
(3rd process)

S74

select Bonding-target P-particle(s) and/or Bonding-target
C-particle(s) to be bound with Each bonding-target F-particle
from Bonding-target P-particle(s) and/or bonding-target
C-particle(s) associated, through 3rd process,
with Concerned bonding-target F-particle
(4th process)

S75

Have all of Bonding-target
P-particles and Bonding-target C-particles
been selected for Bonding-target
F-particles ?

return

**FIG.27**

EP 2 933 744 B1

# FIG.28

EP 2 933 744 B1

FIG.29

# FIG.30

EP 2 933 744 B1

FIG.31

# FIG.32

Embodiment 3 (one C-particle)

Embodiment 2 (five C-particles)

Radial distribution function

Distance between center F-particles

EP 2 933 744 B1

**FIG.33**

Embodiment 4 (high affinity for filler)

Embodiment 2 (normal affinity for filler)

Radial distribution function

Distance between center F-particles

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2672405 A1

**Non-patent literature cited in the description**

- These values were determined by reference to treatise. *J. Chem Phys.,* 1997, vol. 107 (11), 4423-4435

- *Macromolcule,* 2006, vol. 39, 6744